# EUROPÄISCHE PATENTSCHRIFT

(11) **EP 0 600 371 B1**
(45) Veröffentlichungstag und Bekanntmachung des Hinweises auf die Patenterteilung: **03.02.1999**
(21) Anmeldenummer: 93118992.2
(22) Anmeldetag: 25.11.1993
(51) Int. Cl.: C07F 9/38, C07F 9/40, C07F 9/572, A61K 31/66, A61K 31/675

(54) **Guanidinalkyl-1, 1-bisphosphonsäurederivate, Verfahren zu ihrer Herstellung und ihre Verwendung**
Guanidinyl alkyl-1,1- bis phosphonic acid derivatives, process for their preparation and use
Dérivés d'acides guanidinyl alkyl bis-1,1- phosphoniques, procédé pour les préparer et leur utilisation

(30) Priorität: 02.12.1992 DE 4240422; 13.05.1993 DE 4316019; 23.09.1993 DE 4332362
(43) Veröffentlichungstag der Anmeldung: 08.06.1994
(73) Patentinhaber: HOECHST AKTIENGESELLSCHAFT, 65929 Frankfurt am Main (DE)
(72) Erfinder: Naumann, Christoph, Dr., D-65527 Niedernhausen (DE); Lang, Hans-Jochen, Dr., D-65719 Hofhiem/Taunus (DE); Sandow, Jürgen, Dr., D-61479 Glashütten (DE); Moura, Anne-Marie, F-75014 Paris (FR)

(56) Entgegenhaltungen:
- EP-A- 0 010 147
- EP-A- 0 186 405
- EP-A- 0 252 504
- EP-A- 0 546 548
- CHEMICAL ABSTRACTS, vol. 113, no. 9, 27. August 1990, Columbus, Ohio, US; abstract no. 78695b, ISOMURA, Y. ET AL. 'HETEROCYCLE-SUBSTITUTED BIS(PHOSPHONIC ACID) DERIVATIVES AND BONE ABSORPTION INHIBITORS CONTAINING THEM' Seite 812 ; & JP-A-02 048 587 (YAMANOUCHI PHARMACEUTICAL CO., LTD.)

## Beschreibung

Die Therapie von Erkrankungen des Knochensystems gewinnt in zunehmendem Maße an Bedeutung. So ist beispielsweise die Osteoporose eine häufig vorkommende Knochenerkrankung. Bei den verschiedenen Formen der Osteoporose kommt es zu einem starken Verlust an Knochengewebe, so daß schließlich die mechanische Stabilität des Knochens verloren geht. In gesunden Menschen ist die Rate, mit der Osteoclasten und Osteoplasten gebildet werden, so ausgebildet, daß Knochenbildung und Knochenresorption im Gleichgewicht stehen. Bei Osteoporose ist das Gleichgewicht gestört, so daß es zu einem Knochenabbau kommt.

Es ist bereits bekannt, daß sich Guanidinalkyl-1,1-bisphosphonsäurederivate zur Prophylaxe und/oder Behandlung von Osteoporose eignen (EP 0 546 548).

In dem Bestreben, weitere wirksame Verbindungen zur Behandlung und Prophylaxe von Osteoporose mit geringen Nebenwirkungen zu erhalten, wurde nun gefunden, daß die erfindungsgemäßen Guanidinalkyl-1,1-bisphosphonsäuren die Knochenresorption vermindern.

Die Erfindung betrifft daher eine Verbindung der tautomeren Formel Ia, Ib oder Ic 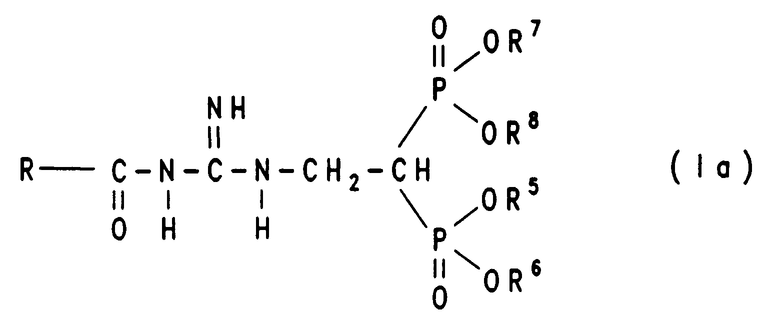 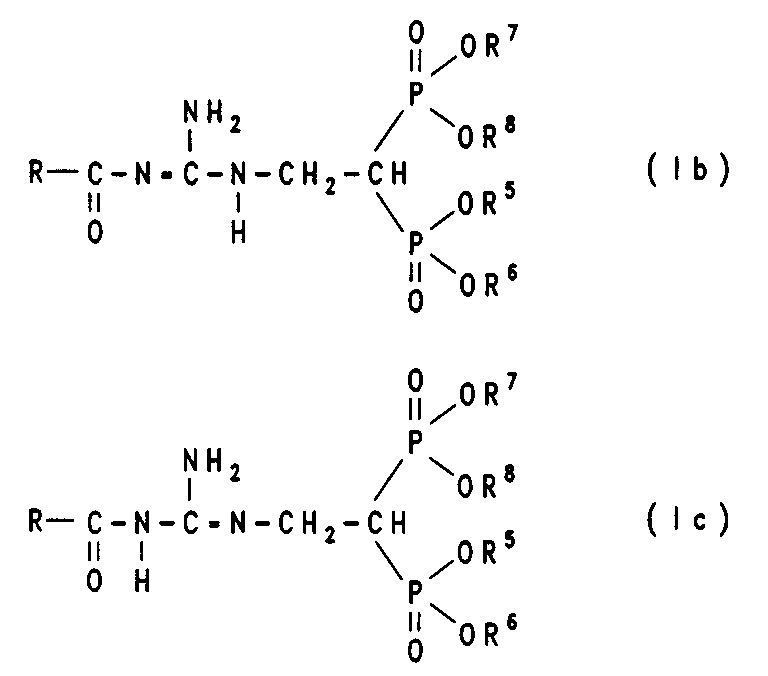 und/oder ein physiologisch verträgliches Salz der Verbindung der Formel Ia, Ib oder Ic;
dabei hat R die folgende Bedeutung:
I) einen Rest der Formel V 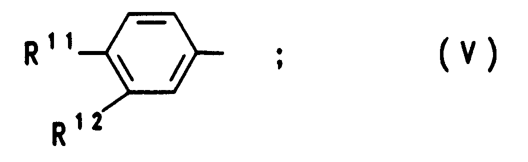 dabei haben R¹¹ oder R¹² die nachstehende Bedeutung:
   a) R¹³ -S(O)ₙ- oder
   b) 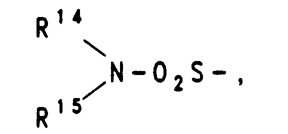 dabei ist R¹³
      1) (C₁-C₆)-Alkyl,
      2) (C₅-C₇)-Cycloalkyl,
      3) Cyclopentylmethyl,
      4) Cyclohexylmethyl,
      5) Phenyl,
      6) Phenyl, ein- bis dreifach substituiert durch
         - 6.1: Fluoratom,
         - 6.2: Chloratom,
         - 6.3: Methyl oder
         - 6.4: Methoxy,
      dabei ist n die ganze Zahl Null, 1 oder 2,
      dabei sind R¹⁴ und R¹⁵ gleich oder verschieden und haben unabhängig voneinander die nachstehende Bedeutung:
      1) Wasserstoffatom,
      2) (C₁-C₆)-Alkyl,
      3) Phenyl,
      4) Phenyl, ein- oder zweifach substituiert durch
         - 4.1: Fluoratom,
         - 4.2: Chloratom,
         - 4.3: Methyl oder
         - 4.4: Methoxy,
      5) -(CH₂)ₘ-Phenyl, wobei m eine ganze Zahl von 1 bis 4 ist, oder
      6) -(CH₂)ₘ-Phenyl, wobei m eine ganze Zahl von 1 bis 4 ist und der Phenylrest ein- oder zweifach substituiert ist durch die in 4.1 bis 4.4 angegebenen Reste,
      7) R¹⁴ und R¹⁵ bilden gemeinsam eine geradkettige oder verzweigte Kette von 4 bis 7 Kohlenstoffatomen, die Kette kann zusätzlich durch
         - 7.1: O,
         - 7.2: S oder
         - 7.3: NR¹⁰
         unterbrochen sein,
         dabei ist
         R¹⁰
         1) Wasserstoffatom oder
         2) Methyl, oder
      8) R¹⁴ und R¹⁵ bilden zusammen mit dem Stickstoffatom, an das sie gebunden sind einen
         - 8.1: Dihydroindol-,
         - 8.2: Tetrahydrochinolin- oder
         - 8.3: Tetrahydroisochinolin-Ring,
         und der jeweils andere Substituent R¹¹ oder R¹² bedeutet
         a) Wasserstoffatom,
         b) Halogenatom, wie Fluor-, Chlor-, Brom- oder Jodatom,
         c) (C₁-C₄)-Alkyl,
         d) (C₁-C₄)-Alkoxy,
         e) Phenoxy,
         f) Phenoxy, ein- bis dreifach substituiert durch
            1) Fluor- oder Chloratom,
            2) Methyl oder
            3) Methoxy,
         g) R¹³-S(O)ₙ, dabei ist n die ganze Zahl Null, 1 oder 2 und R¹³ hat die obengenannte Bedeutung, oder
         h) 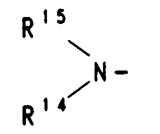 dabei haben R¹⁴ und R¹⁵ die obengenannte Bedeutung, oder
II) einen Rest der Formel VI 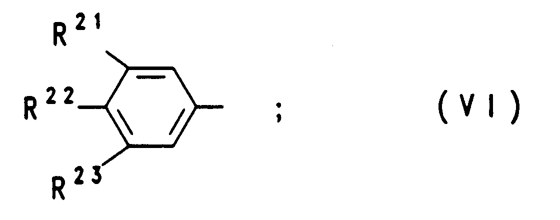 dabei haben R²¹, R²² oder R²³ die nachstehende Bedeutung:
   a) Wasserstoffatom
   b) Halogenatom, wie Fluor-, Chlor-, Brom- oder Jodatom, oder
   c) (C₁-C₁₂)-Alkyl,
   wobei einer der Substituenten R²¹, R²² oder R²³ auch bedeuten kann
   1) N₃,
   2) CN,
   3) OH,
   4) (C₁-C₁₀)-Alkoxy,
   5) R²⁴-CₙH₂ₙ-Oₘ,
      dabei ist
      m die Zahl Null oder 1,
      n ist die Zahl Null, 1, 2 oder 3,
      R²⁴ ist
      1) CₚF₂ₚ₊₁
         dabei ist
         p die Zahl 1, 2 oder 3, sofern
         n die Zahl Null oder 1 ist,
      2) (C₃-C₁₂)-Cycloalkyl,
      3) Phenyl,
      4) Pyridyl,
      5) Chinolyl oder
      6) Isochinolyl,
      wobei das Ringsystem der Reste 3) bis 6) unsubstituiert oder mit einem Rest aus der Gruppe
      - 3.1: Fluoratom,
      - 3.2: Chloratom,
      - 3.3: CF₃,
      - 3.4: Methyl,
      - 3.5: Methoxy oder
      - 3.6: NR²⁵R²⁶
      substituiert ist, dabei sind R²⁵ und R²⁶ gleich oder verschieden und haben unabhängig voneinander die Bedeutung
      - 3.6.1: Wasserstoffatom oder
      - 3.6.2: (C₁-C₄)-Alkyl, oder
III) einen Rest der Formel VII 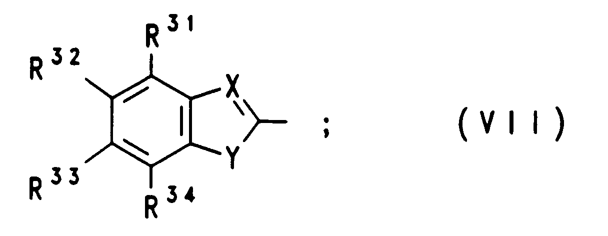 dabei haben R³¹, R³², R³³ oder R³⁴ die nachstehende Bedeutung:
   a) Wasserstoffatom,
   b) Halogenatom, wie Fluor-, Chlor-, Brom- oder Jodatom,
   c) -CN,
   d) -NO₂,
   e) -N₃,
   f) -(C₁-C₆)-Alkyl, geradkettig oder verzweigt oder
   g) R³⁵-CₙH₂ₙ-Z-,
      dabei ist n die Zahl Null, 1, 2, 3, 4, 5 oder 6, und die Alkylenkette -CₙH₂ₙ- ist geradkettig oder verzweigt, und ein C-Atom kann durch ein O-, S- oder N-Atom ersetzt sein,
      R³⁵ ist
      1) Wasserstoffatom,
      2) (C₃-C₆)-Alkenyl,
      3) (C₅-C₈)-Cycloalkyl,
      4) (C₅-C₈)-Cycloalkyl, mit einer Hydroxygruppe substituiert, oder
         eine Methylengruppe ist durch ein O-, S- oder N-Atom ersetzt, oder
      5) Phenyl, unsubstituiert oder substituiert mit 1 bis 3 Resten aus der Gruppe
         - 5.1: Halogenatom, wie Fluor-, Chlor-, Brom- oder Jodatom,
         - 5.2: CF₃,
         - 5.3: CH₃-S(O)ₓ,
         dabei ist x die Zahl Null, 1 oder 2,
         - 5.4: R³⁶-W_{y},
         dabei ist R³⁶ Wasserstoffatom, Methyl oder Ethyl, W ist O-Atom, NH oder NCH₃ und y ist Null oder 1,
         - 5.5: CₘF₂ₘ₊₁,
         dabei ist m die Zahl 1, 2 oder 3,
         - 5.6: Pyridyl,
         - 5.7: Chinolyl oder
         - 5.8: Isochinolyl,
      Z ist
      1) -CO-,
      2) -CH₂-,
      3) -[CH(OH)]_{q}-,
         dabei ist q die Zahl 1, 2 oder 3,
      4) -[C(CH₃)(OH)]_{q}-,
         dabei ist q die Zahl 1, 2 oder 3,
      5) -O-,
      6) -NH-,
      7) 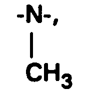
      8) -S(O)ₓ-,
         dabei ist x Null, 1 oder 2,
      9) -SO₂-NH- oder
      10) 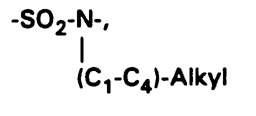
      X hat die nachstehende Bedeutung
      a) N oder
      b) C-R³⁷,
         dabei ist R³⁷ Wasserstoffatom, (C₁-C₄)-Alkyl oder (C₂-C₄)-Alkenyl,
      Y hat die nachstehende Bedeutung
      a) NH,
      b) -N-(C₁-C₆)-Alkyl,
      c) -N-(C₂-C₄)-Alkenyl oder
      d) R³⁵-CₙH₂ₙ-Z-,
         dabei ist R³⁵, n und Z wie unter g) definiert,
      R⁵, R⁶, R⁷ und R⁸ sind gleich oder verschieden und haben unabhängig voneinander die nachstehende Bedetung
      a) Wasserstoffatom,
      b) (C₁-C₅)-Alkyl, geradkettig oder verzweigt, oder
      c) Phenyl.

Enthalten die Substituenten R¹¹, R¹², R²¹, R²², R²³, R²⁴, R²⁵, R²⁶, R³¹, R³², R³³, R³⁴ und R³⁵ ein oder mehrere Asymmetriezentren, so gehören sowohl S-als auch R-konfigurierte Verbindungen zur Erfindung. Die Verbindungen können als optische Isomere, Diastereoisomere, Racemate oder als Gemische derselben vorliegen.
Die bezeichneten Alkylreste können sowohl geradkettig als auch verzweigt vorliegen.

Bevorzugt ist eine Verbindung der Formel Ia, Ib oder Ic, und/oder ein physiologisch verträgliches Salz der Verbindung der Formel Ia, Ib oder Ic, wobei R die folgende Bedeutung hat:
I) einen Rest der Formel V; dabei hat R¹¹ die nachstehende Bedeutung:
   a) Fluoratom,
   b) Chloratom,
   c) 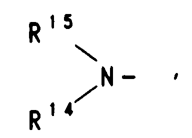 dabei haben R¹⁴ und R¹⁵ die obengenannte Bedeutung,
   d) R¹³-S(O)ₙ-, dabei ist n Null, 1 oder 2, oder
   e) Phenoxy,
   dabei hat R¹² die nachstehende Bedeutung:
   a) R¹³-S(O)ₙ, dabei ist n Null, 1 oder 2, oder
   b) 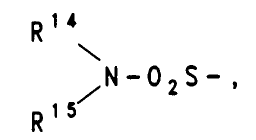 dabei haben R¹⁴ und R¹⁵ die obengenannte Bedeutung, oder
II) einen Rest der Formel VI; dabei haben R²¹, R²² oder R²³ die nachstehende Bedeutung:
   a) Wasserstoffatom,
   b) Halogenatom wie Fluor-, Chlor- oder Bromatom oder
   c) (C₁-C₈)-Alkyl,
   wobei einer der Substituenten R²¹, R²² oder R²³ auch bedeuten kann
   1) OH,
   2) (C₁-C₆)-Alkoxy,
   3) R²⁴-CₙH₂ₙ-Oₘ,
      dabei ist
      m die Zahl Null oder 1,
      n ist die Zahl Null, 1, 2 oder 3,
      R²⁴ bedeutet
      1) CₚF₂ₚ₊₁,
         dabei ist p die Zahl 1, sofern
         n die Zahl Null oder 1 ist,
      2) (C₅-C₇)-Cycloalkyl,
      3) Phenyl,
      4) Pyridyl,
      5) Chinolyl oder 6) Isochinolyl,
      wobei das Ringsystem der Reste 3) bis 6) unsubstituiert oder mit einem Rest aus der Gruppe
      - 3.1: Fluoratom,
      - 3.2: Chloratom,
      - 3.3: CF₃,
      - 3.4: CH₃ oder
      - 3.5: Methoxy
      substituiert ist, oder
III) einen Rest der Formel VII; dabei haben R³¹, R³², R³³ oder R³⁴ die nachstehende Bedeutung:
   a) Wasserstoffatom,
   b) Halogenatom, wie Fluor-, Chlor-, Brom- oder Jodatom,
   c) (C₁-C₆)-Alkyl, geradkettig oder verzweigt, oder
   d) R³⁵-CₙH₂ₙ-Z-,
      dabei ist n die Zahl Null, 1 oder 2, und die Alkylenkette -CₙH₂ₙ- ist geradkettig oder verzweigt, und ein C-Atom kann durch ein O-, S- oder N-Atom ersetzt sein,
      R³⁵ ist
      1) Wasserstoffatom,
      2) (C₅-C₈)-Cycloalkyl,
      3) (C₅-C₈)-Cycloalkyl, mit einer Hydroxygruppe substituiert, oder
         eine Methylengruppe ist durch ein O-, S- oder N-Atom ersetzt, oder
      4) Phenyl, unsubstituiert oder substituiert mit 1 bis 3 Resten aus der Gruppe
         - 4.1: Halogenatom, wie Fluor-, Chlor-, Brom- oder Jodatom,
         - 4.2: CF₃,
         - 4.3: CH₃-S(O)ₓ,
         dabei ist x die Zahl Null, 1 oder 2,
         - 4.4: R³⁶-W_{y},
         dabei ist R³⁶ Wasserstoffatom, Methyl oder Ethyl, W ist O-Atom, NH oder NCH₃ und y ist Null oder 1,
         - 4.5: CₘF₂ₘ₊₁,
         dabei ist m die Zahl 1, 2 oder 3,
         - 4.6: Pyridyl,
         - 4.7: Chinolyl oder
         - 4.8: Isochinolyl,
      Z ist
      1) -CO-,
      2) -CH₂-,
      3) -[CH(OH)]_{q}-,
         dabei ist q die Zahl 1, 2 oder 3,
      4) -[C(CH₃)(OH)]_{q}-,
         dabei ist q die Zahl 1, 2 oder 3,
      5) -O- oder
      6) -S(O)ₓ-,
         dabei ist x Null, 1 oder 2,
      X hat die nachstehende Bedeutung
      a) N oder
      b) CH,
      Y hat die nachstehende Bedeutung
      a) -N-(C₁-C₆)-Alkyl,
      b) -N-(C₂-C₄)-Alkenyl oder
      c) R³⁵-CₙH₂ₙ-Z-,
         dabei ist R³⁵, n und Z wie unter d) definiert,
      R⁵, R⁶, R⁷ und R⁸ sind gleich oder verschieden und haben unabhängig voneinander die nachstehende Bedetung
      a) Wasserstoffatom oder
      b) (C₁-C₅)-Alkyl, geradkettig oder verzweigt.

Besonders bevorzugt ist eine Verbindung der Formel Ia, Ib oder Ic, wobei R¹¹ die folgende Bedeutung hat:
a) 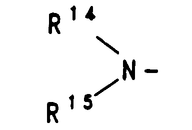 dabei sind R¹⁴ und R¹⁵ gleich oder verschieden und haben unabhängig voneinander die folgende Bedeutung
   1) Wasserstoffatom oder
   2) (C₁-C₄)-Alkyl, oder
   R¹⁴ und R¹⁵ bilden zusammen eine (C₄-C₇)-Alkylkette, oder
b) R¹³-S-,
   dabei ist R¹³
   a) Phenyl, oder
   b) Phenyl, substituiert durch Chlor in para-Position,
dabei hat R¹² die nachstehende Bedeutung:
a) CH₃-SO₂-,
b) H₂N-SO₂-,
c) Phenoxy oder
d) Phenoxy, substituiert durch Chlor in para-Position,
R⁵, R⁶, R⁷ und R⁸ sind gleich oder verschieden und haben unabhängig voneinander die nachstehende Bedeutung
a) Wasserstoffatom oder
b) (C₁-C₄)-Alkyl,
und physiologisch verträgliche Salze der Verbindung der Formel Ia, Ib oder Ic.

Insbesondere bevorzugt sind:
2-[(1-Methyl-2-indolylcarbonyl)-(aminoiminomethyl)amino)]-ethan-1,1-bisphosphonsäuretetraethylester, 2-[(1-Methyl-2-indolylcarbonyl)(aminoiminomethyl)amino)]-ethan-1,1-bisphosphonsäure, 2-[(3-Methylsulfonyl-4-piperidyl-benzoyl)-(aminoiminomethyl)amino)]-ethan-1,1-bisphosphonsäure, 2-[(3-Methylsulfonyl-4-piperidinyl-benzoyl)-(aminoiminomethyl)amino)]-ethan-1,1-bisphosphonsäuretetraethylester, 2-[(3,5-Dichlor-benzoyl)-(aminoiminomethyl)amino)]-ethan-1,1-bisphosphon-säuretetraethylester und 2-[(3,5-Dichlorbenzoyl)-(aminoiminomethyl)amino)]-ethan-1,1-bisphosphonsäure.

Die erfindungsgemäßen Verbindungen lassen sich beispielsweise wie folgt herstellen:

Eine Verbindung der Formel IV 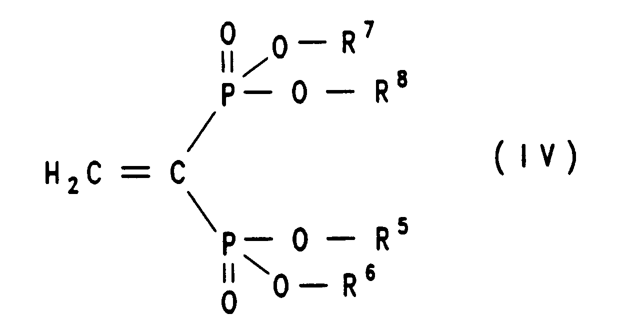 wobei R⁵, R⁶, R⁷ und R⁸ die in Formel Ia genannte Bedeutung haben, wird
A) mit einer Verbindung der Formel III 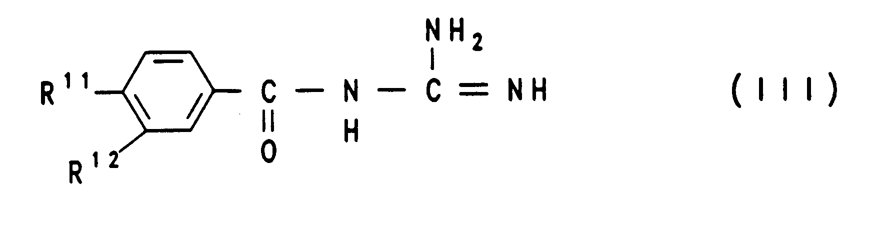 in Gegenwart eines inerten Lösungsmittels zu einer Verbindung der Formel Ia, Ib oder Ic umgesetzt, wobei R¹¹ und R¹² die in Formel Ia, Ib oder Ic genannte Bedeutung haben, und gegebenenfalls wird
B) der Bisphosphonsäureester der Verbindung der Formel Ia, Ib oder Ic in die entsprechende Bisphosphonsäure umgesetzt oder
   eine Verbindung der Formel IV, wobei R⁵, R⁶, R⁷ und R⁸ die in Formel Ia genannte Bedeutung haben, wird
C) mit einer Verbindung der Formel VIII 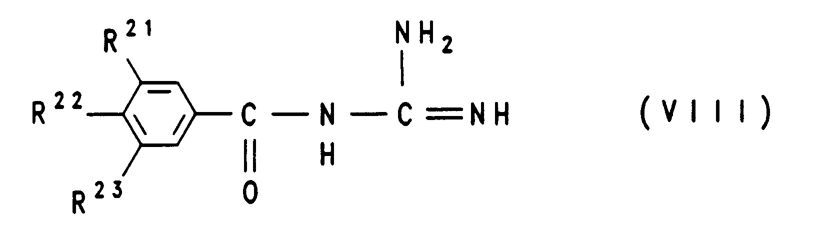 in Gegenwart eines inerten Lösungsmittels zu einer Verbindung der Formel Ia, Ib oder Ic umgesetzt, wobei R²¹, R²² und R²³ die in Formel Ia, Ib oder Ic genannte Bedeutung haben, und gegebenenfalls wird
D) der Bisphosphonsäureester der Verbindung der Formel Ia, Ib oder Ic in die entsprechende Bisphosphonsäure umgesetzt oder
   eine Verbindung der Formel IV, wobei R⁵, R⁶, R⁷ und R⁸ die in Formel Ia genannte Bedeutung haben, wird
E) mit einer Verbindung der Formel IX 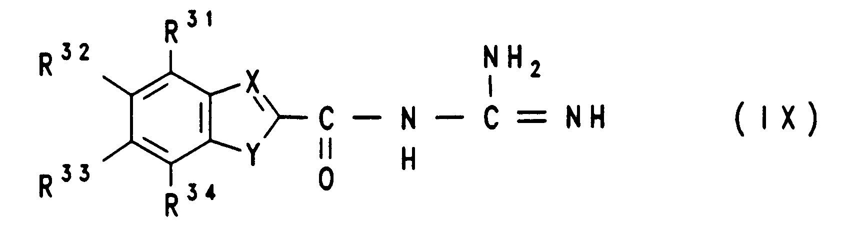 in Gegenwart eines inerten Lösungsmittels zu einer Verbindung der Formel Ia, Ib oder Ic umgesetzt, wobei R³¹, R³², R³³ und R³⁴ die in Formel Ia, Ib oder Ic genannte Bedeutung haben, und gegebenenfalls wird
F) der Bisphosphonsäureester der Verbindung der Formel Ia, Ib oder Ic in die entsprechende Bisphosphonsäure umgesetzt.

Bei der Verfahrensvariante A) geht man am besten so vor, daß man die Verbindung der Formel III in äquimolarer Menge oder in einem bis zu dreifachen Überschuß, gegebenenfalls in einem inerten Lösungsmittel wie Dimethylsulfoxid (DMSO), Dimethylformamid (DMF), Toluol, (C₁-C₄)-Alkanol, Tetrahydrofuran (THF), Dioxan oder Diethylether, mit einer Verbindung der Formel IV unter Zusatz einer Base wie Kaliumcarbonat, Natriumcarbonat, Kaliumhydroxid, Triethylamin, Diethylamin oder wahlweise auch ohne Basenzusatz zu einer Verbindung der Formel Ia, Ib oder Ic umsetzt. Die Reaktionstemperaturen liegen bei etwa 25°C bis 100°C, vorzugsweise bei Verwendung eines Lösungsmittels bei 25°C und dem Siedepunkt des Lösungsmittels, insbesondere bei 70°C. Die Reaktionszeiten betragen 6 bis 48 Stunden, bevorzugt 12 bis 24 Stunden. Die Beendigung der Reaktion kann beispielsweise mittels Dünnschichtchromatographie bestimmt werden.

Zur Isolierung und zur Reinigung der Reaktionsprodukte der Formel Ia, Ib oder Ic kann man die Reaktionsmischung auf einer Kieselgelsäule mit einem Laufmittelgemisch aus Essigester und Alkohol, Volumenverhältnis beispielsweise 6:1, reinigen. Die erhaltenen Verbindungen der Formel Ia, Ib oder Ic können in die korrespondierenden Bisphosphonsäuren durch Hydrolyse (Verfahrensvariante B), z. B. durch Erhitzen unter Reflux in konzentrierter Salzsäure, oder durch Behandlung mit starken Säuren oder Trimethylsilylhalogenid in einem wasserfreien Lösungsmittel und anschließender Hydrolyse erhalten werden. Wasserfreie Bromwasserstoffsäure in Essigsäure kann direkt oder nach entsprechender Verdünnung verwendet werden, oder es wird Trimethylsilyliodid gelöst in einem Lösungsmittel wie Tetrachlorkohlenstoff, Dimethylformamid, Chloroform oder Toluol verwendet. Die Hydrolyse kann unter Kühlung oder Erhitzen ausgeführt werden, z. B. kann der Ester mit einem Trimethylsilylhalogenid unter Kühlung bei -10°C oder darunter umgesetzt werden, und man erhält ein teilweise verseiftes Produkt.

Die Ausgangsverbindungen der Verfahrensvariante A) sind für die Verbindungen III und IV auf einfache Weise nach literaturbekannten Verfahren herstellbar (EP 0 298 553; EP 0 416 499).

Bei der Verfahrensvariante C) geht man am besten so vor, daß man die Verbindung der Formel VIII in äquimolarer Menge oder in einem bis zu dreifachen Überschuß, gegebenenfalls in einem inerten Lösungsmittel wie Dimethylsulfoxid (DMSO), Dimethylformamid (DMF), Toluol, (C₁-C₄)-Alkanol, Tetrahydrofuran (THF), Dioxan oder Diethylether, mit einer Verbindung der Formel IV unter Zusatz einer Base wie Kaliumcarbonat, Natriumcarbonat, Kaliumhydroxid, Triethylamin, Diethylamin oder wahlweise auch ohne Basenzusatz zu einer Verbindung der Formel Ia, Ib oder Ic umsetzt. Die Reaktionstemperaturen liegen bei etwa 25°C bis 100°C, vorzugsweise bei Verwendung eines Lösungsmittels zwischen etwa 25°C und dem Siedepunkt des Lösungsmittels, insbesondere bei 70°C. Die Reaktionszeiten betragen 6 bis 48 Stunden, bevorzugt 12 bis 24 Stunden. Die Beendigung der Reaktion kann beispielsweise mittels Dünnschichtchromatographie bestimmt werden.

Zur Isolierung und zur Reinigung der Reaktionsprodukte der Formel Ia, Ib oder Ic kann man die Reaktionsmischung auf einer Kieselgelsäule mit einem Laufmittelgemisch aus Essigester und Alkohol, Volumenverhältnis beispielsweise 6:1, reinigen. Die erhaltenen Verbindungen der Formel Ia, Ib oder Ic können in die korrespondierenden Bisphosphonsäuren durch Hydrolyse (Verfahrensvariante D), z. B. durch Erhitzen unter Reflux in konzentrierter Salzsäure, oder durch Behandlung mit starken Säuren oder Trimethylsilylhalogenid in einem wasserfreien Lösungsmittel und anschließende Hydrolyse erhalten werden. Wasserfreie Bromwasserstoffsäure in Essigsäure kann direkt oder nach entsprechender Verdünnung verwendet werden, oder es wird Trimethylsilyliodid gelöst in einem Lösungsmittel wie Tetrachlorkohlenstoff, Dimethylformamid, Chloroform oder Toluol verwendet. Die Hydrolyse kann unter Kühlung oder Erhitzen ausgeführt werden, z. B. kann der Ester mit einem Trimethylsilylhalogenid unter Kühlung bei -10°C oder darunter umgesetzt werden, und man erhält ein teilweise verseiftes Produkt.

Die Ausgangsverbindungen der Verfahrensvariante C) sind für die Verbindung der Formel IV auf einfache Weise nach literaturbekannten Verfahren herstellbar (EP 0 298 553; EP 0 416 499).

Die Verbindungen der Formel VIII lassen sich dadurch herstellen, daß man eine Verbindung der Formel II 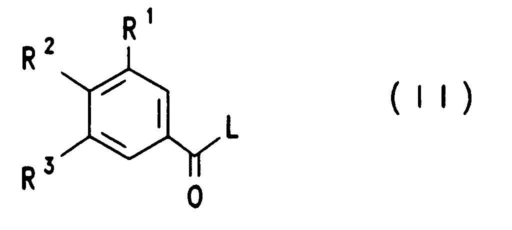 mit Guanidin umsetzt, worin R¹ bis R³ die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.
Neben den Carbonsäurechloriden der Formel II (L = Cl) lassen sich auch weitere aktivierte Säurederivate der Formel II in an sich bekannter Weise direkt aus den zugrundeliegenden Benzoesäure-Derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol (L = 1-Imidazolyl, Staab, Angew. Chem. Int. Ed. Eng. 1, 351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluoborat ("TOTU")(Weiss und Krommer, Chemiker Zeitung 98, 817 (1974)). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol oder THF zwischen 20°C und Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Benzoesäure-Derivate der Formel II sind bekannt und in der Literatur beschrieben. Die unbekannten Verbindungen der Formel II können nach literaturbekannten Methoden hergestellt werden.

Carbonsäuren oder deren Ester der Formel II (z. B. L = -OH oder -O-Methyl) mit R² in der Bedeutung von Halogen oder R³ in der Bedeutung von Nitro können als vielseitige Ausgangsverbindungen für weitere Carbonsäuren der Formel II dienen, wobei das Halogen in der Position R² sehr bequem in bekannter Weise gegen zahlreiche nucleophile Reagenzien, wie Phenole oder Alkohole R⁴-CₙH₂ₙ-OH bzw. deren Alkalisalze unter Bildung entsprechender Benzoesäure-Derivate ausgetauscht werden. Ebenso können Nitrogruppen nach Reduktion zur entsprechenden Aminobenzoesäure durch Sandmeier- oder Ullmann-Reaktionen zu gewünschten, insbesondere halogen-substituierten Benzoesäure-Derivaten führen. Chlor, Brom oder Iod kann in vielen Fällen auch durch direkte Halogenierung mit Hilfe eines Friedel-Crafts-Katalysators in an sich bekannter Weise in eine jeweilige Benzoesäure eingeführt werden.

Bei der Verfahrensvariante E) geht man am besten so vor, daß man die Verbindung der Formel IX in äquimolarer Menge oder in einem bis zu dreifachen Überschuß, gegebenenfalls in einem inerten Lösungsmittel wie Dimethylsulfoxid (DMSO), Dimethylformamid (DMF), Toluol, (C₁-C₄)-Alkanol, Tetrahydrofuran (THF), Dioxan oder Diethylether, mit einer Verbindung der Formel IV unter Zusatz einer Base wie Kaliumcarbonat, Natriumcarbonat, Kaliumhydroxid, Triethylamin, Diethylamin oder wahlweise auch ohne Basenzusatz zu einer Verbindung der Formel Ia, Ib oder Ic umsetzt. Die Reaktionstemperaturen liegen bei 25°C bis 100°C, vorzugsweise bei Verwendung eines Lösungsmittels liegen die Reaktionstemperaturen von 25°C bis zum Siedepunkt des Lösungsmittels, insbesondere um 70°C. Die Reaktionszeiten betragen 6 bis 48 Stunden, bevorzugt 12 bis 24 Stunden. Die Beendigung der Reaktion kann beispielsweise mittels Dünnschichtchromatographie bestimmt werden.

Zur Isolierung und zur Reinigung der Reaktionsprodukte der Formel Ia, Ib oder Ic kann man die Reaktionsmischung auf einer Kieselgelsäule mit einem Laufmittelgemisch aus Essigester und/oder Alkohol, Volumenverhältnis beispielsweise 6:1, reinigen. Die erhaltenen Verbindungen der Formel Ia, Ib oder Ic können in die korrespondierenden Bisphosphonsäuren durch Hydrolyse (Verfahrensvariante F), z. B. durch Erhitzen unter Reflux in konzentrierter Salzsäure, oder durch Behandlung mit starken Säuren oder Trimethylsilylhalogenid in einem wasserfreien Lösungsmittel und anschließende Hydrolyse erhalten werden. Wasserfreie Bromwasserstoffsäure in Essigsäure kann direkt oder nach entsprechender Verdünnung verwendet werden, oder es wird Trimethylsilyliodid gelöst in einem Lösungsmittel wie Tetrachlorkohlenstoff, Dimethylformamid, Chloroform oder Toluol verwendet. Die Hydrolyse kann unter Kühlung oder Erhitzen ausgeführt werden, z. B. kann der Ester mit einem Trimethylsilylhalogenid unter Kühlung bei -10°C oder darunter umgesetzt werden, und man erhält ein teilweise verseiftes Produkt.

Die Ausgangsverbindungen der Verfahrensvariante A) sind für die Verbindung der Formel IV auf einfache Weise nach literaturbekannten Verfahren herstellbar (EP 0 298 553; EP 0 416 499).

Die Verbindungen der Formel IX lassen sich auf bekannte Weise herstellen (DE 43 26 005.5).

Die Erfindung betrifft auch Arzneimittel, die mindestens eine wirksame Menge von mindestens einer Verbindung der Formel Ia, Ib oder Ic und/oder mindestens eines der physiologisch verträglichen Salze einer Verbindung der Formel Ia, Ib oder Ic enthält, neben pharmazeutisch geeigneten und physiologisch verträglichen Hilfs- und Trägerstoffen, Verdünnungsmitteln und/oder anderen Wirkstoffen.

Die Erfindung betrifft ferner die Verwendung der Verbindungen der Formel Ia, Ib oder Ic und/oder deren physiologisch verträglichen Salzen zur Herstellung eines Arzneimittels zur Prophylaxe und Behandlung von degenerativen Erkrankungen des Knochensystems.

Die Erfindung betrifft auch die Verwendung der Verbindungen der Formel Ia, Ib oder Ic und/oder deren physiologisch verträglichen Salze zur Herstellung eines Arzneimittels zur Behandlung von Erkrankungen mit erhöhter Knochenresorption, insbesondere metastatischer Osteokarcinoma, Paget-Disease, Hypercalcemia oder Osteoporose.

Die erfindungsgemäßen Arzneimittel können oberflächlich, percutan, nasal, intravenös, intramuskulär, intraperitoneal, subcutan, intraartikulär, periartikulär, rektal oder oral verabreicht werden.

Die Herstellung der erfindungsgemäßen Arzneimittel zur Prophylaxe und Behandlung von Osteoporose erfolgt, indem mindestens eine Verbindung der Formel Ia, Ib oder Ic und/oder eines von deren physiologisch verträglichen Salzen gegebenenfalls mit Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblicher Hilfsstoffe.

Die Verbindung der Formel Ia, Ib oder Ic kann dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subcutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel Ia, Ib, oder Ic in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von 0,1 bis 10, insbesondere von 0,3 bis 3 Gew.-%.

Die Dosierung des zu verabreichenden Wirkstoffs der Formel Ia, Ib oder Ic und die Häufigkeit der Verabreichung hängen von der Wirkstärke und Wirkdauer der verwendeten Verbindung ab; außerdem auch von Art und Stärke der zu behandelnden Krankheit sowie von Geschlecht, Alter, Gewicht und individueller Ansprechbarkeit des zu behandelnden Säugetiers oder Menschen.

Die anzuwendende Dosierung der erfindungsgemäßen Arzneimittel ist abhängig von verschiedenen Faktoren wie Darreichungsform des Medikaments und Zustand, Gewicht und Schwere der Erkrankung des Patienten. Eine Tagesdosis von ca. 5000 mg der erfindungsgemäßen Arzneimittel sollte jedoch nur kurzzeitig überschritten werden. Bevorzugt sind 10 bis 2500 mg als Tagesdosis für einen Menschen von 70 kg Körpergewicht. Die Verabreichung der Tagesdosis der erfindungsgemäßen Arzneimittel kann in Form einer einzelnen Verabreichung oder in mehreren kleinen Dosen erfolgen. Bevorzugt ist die Verabreichung in 3 bis 8 Dosen pro Tag.

Nachfolgend ist die Erfindung an Hand von Beispielen näher erläutert. Die Prozentangaben beziehen sich auf Volumenprozente, falls nicht anders angegeben.

### Beispiel 1:

### Herstellung von 2-[(3-methylsulfonyl-4-piperidyl-benzoyl)-(aminoiminomethyl)amino]-ethan-1,1-bisphosphonsäuretetraethylester

3,24 g (10 mMol) (3-Methylsulfonyl-4-piperidyl-benzoyl)-(aminoiminomethyl)amin und 3,0 g (10 mMol) Vinyldiphosphonsäuretetraethylester werden in 40 ml absolutem Tetrahydrofuran gelöst. Hierzu gibt man 0,5 g (3,6 mMol) trockenes Kaliumcarbonat und erhitzt 3,5 Stunden unter Schutzgas zum Sieden. Anschließend werden erneut 0,33 g (2,4 mMol) Kaliumcarbonat zugegeben, und man erhitzt weitere 2 Stunden zum Sieden. Anschließend wird das Reaktionsgemisch weitere 16 Stunden bei Raumtemperatur gerührt.
Nach Abfiltrieren und Abtrennen des Lösungsmittels erhält man 4,5 g Rohsubstanz. Die Substanz wird über Kieselgelsäule chromatographiert. Als Laufmittel dient Essigester mit 10 % Ethanol.
Ausbeute: 3,2 g (52 % d.Th.)
Schmelzpunkt: 148 bis 152°C
³¹P-NMR-Spektroskopie:
   - (CDCl₃): δP = 21,77 ppm

### Beispiel 2:

### Herstellung von 2-[(3-Methylsulfonyl-4-piperidyl-benzoyl)-(aminoiminomethyl)amino]-ethan-1,1-bisphosphonsäure

1,5 g (2,4 mMol) 2-[(3-Methylsulfonyl-4-piperidyl-benzoyl)-(aminoiminomethyl)amino)]-ethan-1,1-bisphosphonsäuretetraethylester aus Beispiel 1 werden in 60 ml absolutem Dioxan bei 60°C gelöst. Anschließend läßt man die Reaktionslösung auf Raumtemperatur kommen und gibt 1,64 g (10,8 mMol) Bromtrimethylsilan zu. Man rührt 16 Stunden bei Raumtemperatur und erwärmt anschließend weitere 4 Stunden auf 60°C.
Anschließend werden Lösungsmittel und Leichtsieder bei 0,1 Torr/40°C abgetrennt. Zu dem Rückstand werden dann 15 ml Wasser gegeben, und das Gemisch wird 4 Stunden bei Raumtemperatur gerührt. Der entstandene Rückstand wird abfiltriert und mehrmals mit Ethanol gewaschen. Anschließend wird der hierbei erhaltene Rückstand in 20 ml Methanol und 10 ml Wasser aufgekocht.
Ausbeute: 460 mg (38 % d.Th.)
Schmelzpunkt: 209°C
³¹P-NMR-Spektroskopie:
   - (NaOD/D₂O): 19,13 ppm.

### Beispiel 3:

### Herstellung von 2-[(3,5-Dichlorbenzoyl)-(aminoiminomethyl)amino)]-ethan-1,1-bisphosphon-säure-tetra-ethylester

650 mg (2,4 mMol) (3,5-Dichlor-benzoyl)-(aminoiminomethyl)amin und 730 mg (2,4 mMol) Vinyldiphosphonsäuretetraethylester werden in 30 ml Toluol und 6 ml Dimethylformamid (DMF) gelöst. Hierzu gibt man 0,17 g (2,4 mMol) trockenes Kaliumcarbonat und erhitzt 21 Stunden zum Sieden.
Anschließend wird die Reaktionslösung filtriert und einrotiert. Man erhält 1,8 g Rohprodukt. Die Substanz wird über eine Kieselgelsäule gereinigt. Als Laufmittel dient Aceton.
Ausbeute: 450 mg (35% d.Th.)
Schmelzpunkt: 146 bis 149°C
³¹P-NMR-Spektroskopie:
   - (CDCl₃): 21,73 ppm

### Beispiel 4:

### Herstellung von 2-[(3,5-Dichlor-benzoyl)-(aminoiminomethyl)amino)]-ethan-1,1-bisphosphon-säure

350 mg (0,62 mMol) 2-[(3,5-Dichlor-benzoyl)-(aminoiminomethyl)amino)]-ethan-1,1-bisphosphon-säuretetraethylester werden in 20 ml Acetonitril gelöst. Hierzu gibt man unter Argon 0,3 g Natriumiodid und 0,41 ml (3,10 mMol) Bromtrimethylsilan. Man rührt 16 Stunden bei Raumtemperatur und anschließend 1 Stunde bei 40°C. Anschließend wird das gebildete Natriumbromid abfiltriert und die Mutterlauge vom Lösungsmittel und Leichtsiedern bei 0,1 Torr/40°C abgetrennt. Zu dem Rückstand gibt man 20 ml Methylenchlorid und saugt erneut von gebildetem Feststoff ab. Das Methylenchlorid wird bei 0,1 Torr/40°C abgetrennt.
Anschließend werden 15 ml Wasser zugesetzt und 15 Minuten bei Raumtemperatur gerührt. Das Wasser wird bei 12 Torr/60°C entfernt. Zu dem erhaltenen Rückstand gibt man dann 10 ml Isopropanol und setzt ca 1 bis 2 ml Wasser hinzu. Das Ganze wird 5 Minuten erhitzt. Nach dem Abkühlen saugt man die farblosen Kristalle ab und trocknet im Vakuum bei 12 Torr.
Ausbeute: 170 mg (60%)
Schmelzpunkt: 267°C
³¹P-NMR-Spektroskopie:
   - (NaOD/D₂O): δ³¹ P = 19,09 ppm
¹H-NMR-Spektroskopie:
   - (NaOD/D₂O): δ = 1,90 (tt, ²J_{PH} = 14 Hz, ³J_{HH} = 6 Hz, 1 H); 3,48 (td, ³J_{PH} = 14 Hz, ³J_{HH} = 6 Hz, 2H); 7,45 (s, 1H); 7,54 (s2H).
¹³C-NMR-Spektroskopie:
   - (NaOD/D₂O): δ = 43,8 (C-1); 44,1 (C-2); 129,9 (C-3'); 133,0 (C-4); 136,7 (C-5,C-5'); 142,0 (C-6); 162,5 (C-Gua); 174,9 (C=O).
MS-Spektroskopie:
m/e = 419

### Beispiel 5:

### Herstellung von 2-[(1-Methyl-2-indolylcarbonyl)-(aminoiminomethyl)amino)]-ethan-1,1-bisphosphonsäure-tetra-ethylester

1,0 g (3,9 mMol) (1-Methyl-2-indolylcarbonyl-guanidin-hydrochlorid) und 1,2 g (4 mMol) Vinyldiphosphonsäuretetraethylester werden in 40 ml Tetrahydrofuran (THF) und 15 ml Dimethylformamid (DMF) gelöst. Hierzu gibt man 0,55 g (4 mMol) trockenes Kaliumcarbonat und erhitzt 21 Stunden zum Sieden. Anschließend wird die Reaktionslösung filtriert und einrotiert. Man erhält 2,0 g Rohprodukt. Die Substanz wird über eine Kieselgelsäule gereinigt. Als Laufmittel dient Ethanol.
Ausbeute: 1,4g (70 % d.Th.)
³¹P-NMR-Spektroskopie:
   - (CDCl₃): δ = 21,98 ppm

### Beispiel 6:

### Herstellung von 2-[(1-Methyl-2-indolylcarbonyl)-(aminoiminomethyl)amino)]-ethan-1,1-bisphosphonsäure

1 g (1,8 mMol) 2-[(1-Methyl-2-indolylcarbonyl]-(aminoiminomethyl)amino)]-ethan-1,1-bisphosphonsäure-tetraethylester aus Beispiel 5 werden in 40 ml Acetonitril gelöst. Hierzu gibt man unter Argon 0,6 g Natriumjodid und 1,2 ml (90 mMol) Bromtrimethylsilan. Man rührt 16 Stunden bei Raumtemperatur und anschließend 1 Stunde bei 40°C. Anschließend wird das gebildete Natriumbromid abfiltriert und die Mutterlauge vom Lösungsmittel und Leichtsiedern bei 0,1 Torr/40°C abgetrennt. Zu dem Rückstand gibt man 20 ml Methylenchlorid und saugt erneut von gebildetem Feststoff ab. Das Methylenchlorid wird bei 0,1 Torr/40°C abgetrennt.
Anschließend werden 15 ml Wasser zugesetzt und 15 Minuten bei Raumtemperatur gerührt. Das Wasser wird bei 12 Torr/60°C entfernt. Zu dem erhaltenen Rückstand gibt man dann 10 ml Isopropanol und setzt ca. 1 bis 2 ml Wasser hinzu. Das Ganze wird 5 Minuten erhitzt. Nach dem Abkühlen saugt man die farblosen Kristalle ab und trocknet bei 12 Torr.
Ausbeute: 300 mg (38 %)
³¹P-NMR-Spektroskopie:
   - (NaOD/D₂O): δ³¹P = 18,07 ppm
¹H-NMR-Spektroskopie:
   - (NaOD/D₂O): δ = 1,98 (tt, ²J_{PH} = 14 Hz, ³J_{HH} = 6 Hz, 1 H); 3,65 (td, ³J_{PH} = 14 Hz, ³J_{HH} = 6 Hz, 2 H); 7,08 bis 8,56 (Aromaten-H, 5).
¹³C-NMR-Spektroskopie:
   - (NaOD/D₂O): δ = 32,5 (C-1); 41,8 (C-2); 41,9 (C-3); 103,2 (C-4); 105,2 (C-5); 120,2 (C-6); 122,0 (C-7); 124,1 (C-8); 126,2 (C-9); 137,8 (C-10); 139,2 (C-11); 139,7 (C-12); 160,8 (C-Gua); 172,2 (C = O)
MS-Spektroskopie:
m/e = 419

### Beispiel 7:

### Herstellung von 2-[(3-Methylsulfonyl-4-phenoxy-benzoyl)-(aminoiminomethyl)amino]-ethan-1,1-bisphosphonsäuretetraethylester

1,8 g (4,86 mMol) (3-Methylsulfonyl-4-phenoxy-benzoyl)-(aminoiminomethyl)amin und 1,46 g (4,86 mMol) Vinyldiphosphonsäuretetraethylester werden wie in Beispiel 1 beschrieben umgesetzt.

Die Substanz wird über eine Kieselgelsäure chromatographiert. Als Laufmittel dient Essigester:Aceton = 1:1.
Ausbeute: 800 mg (26 %)
Schmelzpunkt: 145°C
³¹P-NMR-Spektroskopie:
   - (CDCl₃): δ³¹P = 22,10 ppm
¹H-NMR-Spektroskopie:
   - (CDCl₃): δ = 1,35 (t, 12 H); 3,31 (s, 3 H); 4,0 (mc, 2 H); 4,20 (mc, 9 H); 6,88 (d, 1 H); 7,13 (mc, 2 H); 7,24 (mc, 1 H); 7,42 (mc, 2 H); 8,35 (d, 1 H); 8,89 (s, 1 H) ppm.
¹³C-NMR-Spektroskopie:
   - (CDCl₃): δ = 16,43; 29,30; 37,89; 43,36; 53,87; 63,14; 117,25; 120,36; 125,33; 129,72; 130,25; 155,06; 157,82; 175,0 ppm.

### Beispiel 8

### Herstellung von 2-[(3-Methylsulfonyl-4-N,N-diethylamino-benzoyl)-(aminoiminomethyl)amino]-ethan-1,1-bisphosphonsäuretetraethylester

1,0 g (2,9 mMol) (3-Methylsulfonyl-4-N,N-diethylamino-benzoyl)-(aminoiminomethyl]amin und 0,9 g (2,9 mMol) Vinyldiphosphonsäuretetraethylester werden wie in Beispiel 1 beschrieben umgesetzt.

Die Substanz wird über eine Kieselgelsäure chromatographiert. Als Laufmittel dient Essigester:Aceton = 1:1.
Ausbeute: 1,2 g (66,6 %)
Schmelzpunkt: 121°C
³¹P-NMR-Spektroskopie:
   - (CDCl₃): δ³¹P = 22,12 ppm
¹H-NMR-Spektroskopie:
   - (CDCl₃): δ = 1,07 (t, 6 H); 1,35 (t, 12 H); 3,18 (q, 4 H); 3,34 (s, 3 H); 3,98 (mc, 2 H); 4,20 (mc, 9 H); 7,32 (d, 1 H); 8,36 (d, 1 H); 8,92 (s, 1 H) ppm.
¹³C-NMR-Spektroskopie:
   - (CDCl₃): δ = 12,09; 16,38; 38,20; 42,59; 48,37; 63,34; 124,46; 137,21; 136,99; 153,34; 175,64 ppm.

### Beispiel 9

### Herstellung von 2-[(3-Methylsulfonyl-4-phenoxy-benzoyl)-(aminoiminomethyl)amino]-ethan-1,1-bisphosphonsäure

350 mg (0,55 mMol) 2-[(3-Methylsulfonyl-4-phenoxy-benzoyl)-(aminoiminomethyl)amino]-ethan-1,1-bisphosphonsäuretetraethylester aus Beispiel 7 werden wie in Beispiel 2 beschrieben umgesetzt.

Das Produkt wird nach der Hydrolyse mit 40 ml Wasser abgesaugt und mit 30 ml Aceton gewaschen.
Ausbeute: 150 mg (52,4 %)
Schmelzpunkt: 242°C
³¹P-NMR-Spektroskopie:
   - (NaOD/D₂O): δ³¹ P = 17,65 ppm
¹H-NMR-Spektroskopie:
   - (NaOD/D₂O): δ = 2,04 (tt, 1 H); 3,50 (s, 1 H); 3,65 (td, 2 H); 7,06 (d, 1 H); 7,26 (d, 2 H); 7,38 (mc, 1 H); 7,54 (mc, 2 H); 8,20 (mc, 1 H); 8,55 (m, 1 H) ppm.

### Beispiel 10

### Herstellung von 2-[(3-Methylsulfonyl-4-N,N-diethylamino-benzoyl)-(aminoiminomethyl)amino]-ethan-1,1-bisphosphonsäure

600 mg (0,92 mMol) 2-[(3-Methylsulfonyl-4-N,N-diethylamino-benzoyl)-(aminoiminomethyl)-amino)]-ethan-1,1-bisphosphonsäuretetraethylester aus Beispiel 8 werden wie in Beispiel 2 beschrieben umgesetzt.

Nach der Hydrolyse mit 30 ml Wasser wird das Produkt abgesaugt und anschließend mit Aceton versetzt.
Ausbeute: 483 mg (100 %)
Schmelzpunkt: 181°C
³¹P-NMR-Spektroskopie:
   - (NaOD/D₂O): δ³¹P = 17,70 ppm (²J_{PH} = 20,9 Hz; ³J_{PH} = 14,2 Hz)
¹H-NMR-Spektroskopie:
   - (NaOD/D₂O): δ = 1,03 (t, 6 H); 2,11 (tt, 1 H); 3,12 (q, 4 H); 3,53 (s, 3 H); 3,65 (td, 2 H); 7,58 (d, 1 H); 8,26 (mc, 1 H); 8,58 (m, 1 H) ppm.

### Beispiel 11:

### Die Wirksamkeit der erfindungsgemäßen Verbindungen wird in vitro in folgenden Versuchen nachgewiesen.

Die Knochenresorption wird anhand der Analyse von ⁴⁵Ca-Freisetzung aus Schädeldecken von fötalen Ratten, die 20 Tage alt waren, bestimmt. Die Knochen werden markiert indem trächtigen Ratten 200 µCi/kg ⁴⁵CaCl₂ injiziert wird, 2 Tage bevor die Schädeldecke der Föten seziert wird.

### 1. Kultivierung der Knochen

Die Schädeldecke der Föten wird in zwei Hälften geteilt. Eine Schädeldeckenhälfte dient der Kontrolle, die andere Hälfte wird mit den erfindungsgemäßen Verbindungen inkubiert.
Jede Schädelhälfte wird in einer sterilen Plastikschale kultiviert. Das Kultivierungsmedium (BGJb-Medium, Gibco) enthält je ml 10 % fötales Kälberserum, Penicillin-Streptomycin (100 000 Units/l, Gibco) und Ascorbinsäure (50 mg/l). Die Schädelhälften werden bei 37°C, in einer Gasatmosphäre von 5 % CO₂ und 95% O₂ inkubiert. Nach 48 Stunden wird das Kultivierungsmedium durch frisches ersetzt, die erfindungsgemäßen Verbindungen und Parathyroid-Hormon (10⁻⁷M) werden zugefügt und weitere 48 Stunden inkubiert. Der Kontrolle wird Parathyroid. Hormon (10⁻⁷ M Sigma) zugefügt.
Am Ende des Expiriments wird die Aktivität von ⁴ ⁵Ca im Kulturmedium und im Knochen bestimmt.
Die Ergebnisse in Tabelle 1 zeigen die Inhibierung der ⁴ ⁵Ca-Freisetzung ins Kulturmedium in Prozent. Die Ergebnisse sind der Durchschnittswert von 3 bis 5 Experimenten.

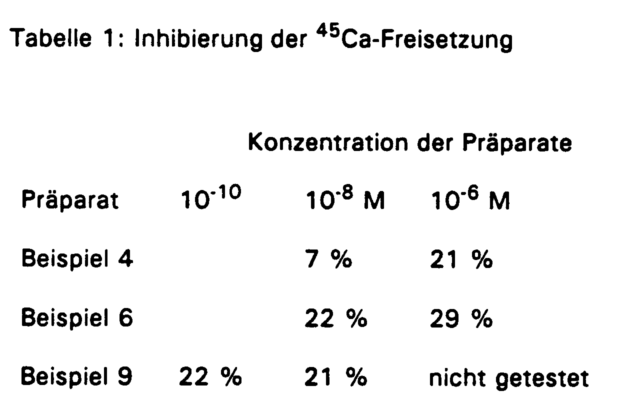

## Patentansprüche

1. Verbindung der tautomeren Formel Ia, Ib oder Ic 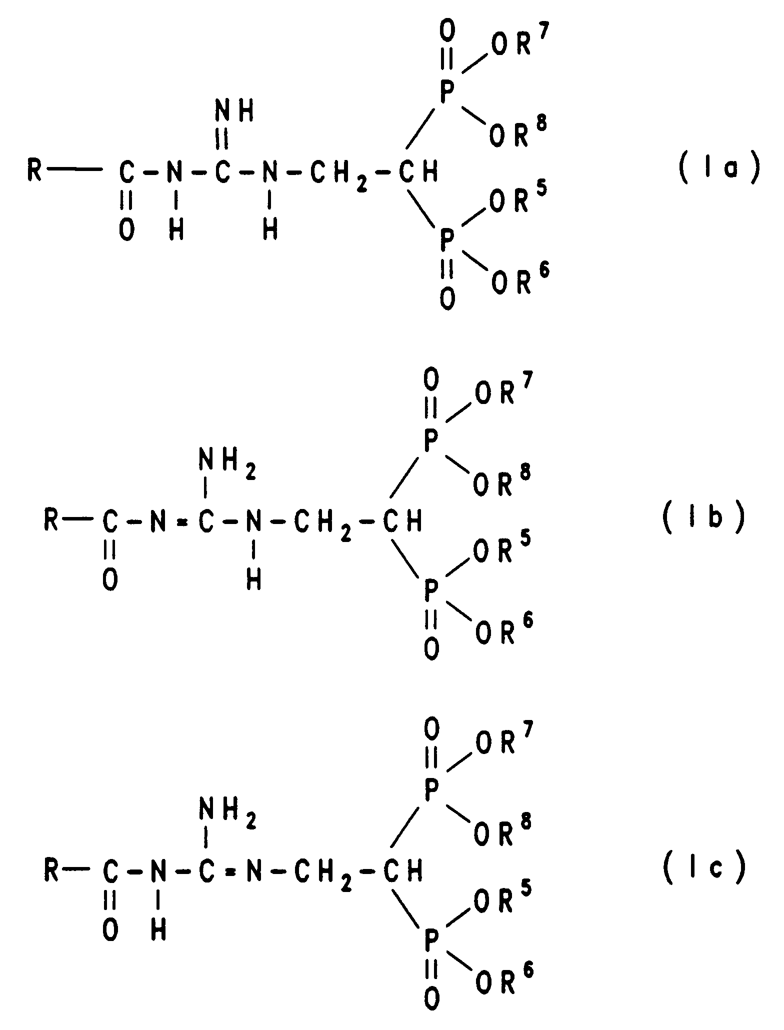 und/oder ein physiologisch verträgliches Salz der Verbindung der Formel Ia, Ib oder Ic;
dabei hat R die folgende Bedeutung:
I) einen Rest der Formel V 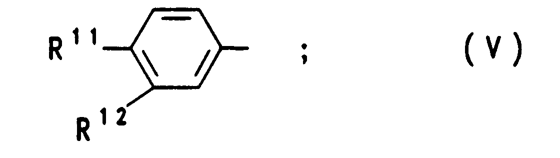 dabei haben R¹¹ oder R¹² die nachstehende Bedeutung:
a) R¹³ -S(O)ₙ- oder
b) 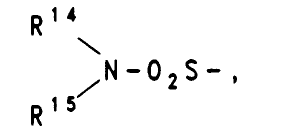 dabei ist R¹³
1) (C₁-C₆)-Alkyl,
2) (C₅-C₇)-Cycloalkyl,
3) Cyclopentylmethyl,
4) Cyclohexylmethyl,
5) Phenyl,
6) Phenyl, ein- bis dreifach substituiert durch
6.1 Fluoratom,
6.2 Chloratom,
6.3 Methyl oder
6.4 Methoxy,
dabei ist n die ganze Zahl Null, 1 oder 2,
dabei sind R¹⁴ und R¹⁵ gleich oder verschieden und haben unabhängig voneinander die nachstehende Bedeutung:
1) Wasserstoffatom,
2) (C₁-C₆)-Alkyl,
3) Phenyl,
4) Phenyl, ein- oder zweifach substituiert durch
4.1 Fluoratom,
4.2 Chloratom,
4.3 Methyl oder
4.4 Methoxy,
5) -(CH₂)ₘ-Phenyl, wobei m eine ganze Zahl von 1 bis 4 ist, oder
6) -(CH₂)ₘ-Phenyl, wobei m eine ganze Zahl von 1 bis 4 ist und der Phenylrest ein- oder zweifach substituiert ist durch die in 4.1 bis 4.4 angegebenen Reste,
7) R¹⁴ und R¹⁵ bilden gemeinsam eine geradkettige oder verzweigte Kette von 4 bis 7 Kohlenstoffatomen, die Kette kann zusätzlich durch
7.1 O,
7.2 S oder
7.3 NR¹⁰
unterbrochen sein,
dabei ist
R¹⁰
1) Wasserstoffatom oder
2) Methyl, oder
8) R¹⁴ und R¹⁵ bilden zusammen mit dem Stickstoffatom, an das sie gebunden sind einen
8.1 Dihydroindol-,
8.2 Tetrahydrochinolin- oder
8.3 Tetrahydroisochinolin-Ring,
und der jeweils andere Substituent R¹¹ oder R¹² bedeutet
a) Wasserstoffatom,
b) Halogenatom, wie Fluor-, Chlor-, Brom- oder Jodatom,
c) (C₁-C₄)-Alkyl,
d) (C₁-C₄)-Alkoxy,
e) Phenoxy,
f) Phenoxy, ein- bis dreifach substituiert durch
1) Fluor- oder Chloratom,
2) Methyl oder
3) Methoxy,
g) R¹³-S(O)ₙ, dabei ist n die ganze Zahl Null, 1 oder 2 und R¹³ hat die obengenannte Bedeutung, oder
h) 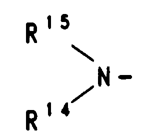 dabei haben R¹⁴ und R¹⁵ die obengenannte Bedeutung, oder
II) einen Rest der Formel VI 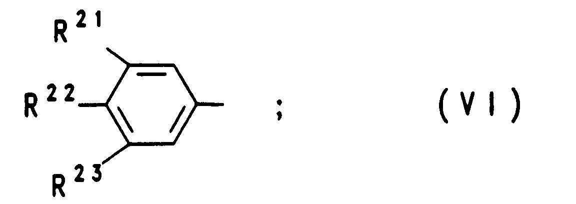 dabei haben R²¹, R²² oder R²³ die nachstehende Bedeutung:
a) Wasserstoffatom
b) Halogenatom, wie Fluor-, Chlor-, Brom- oder Jodatom, oder
c) (C₁-C₁₂)-Alkyl,
wobei einer der Substituenten R²¹, R²² oder R²³ auch bedeuten kann
1) N₃,
2) CN,
3) OH,
4) (C₁-C₁₀)-Alkoxy,
5) R²⁴-CₙH₂ₙ-Oₘ,
dabei ist
m die Zahl Null oder 1,
n ist die Zahl Null, 1, 2 oder 3,
R²⁴ ist
1) CₚF₂ₚ₊₁
dabei ist
p die Zahl 1, 2 oder 3, sofern
n die Zahl Null oder 1 ist,
2) (C₃-C₁₂)-Cycloalkyl,
3) Phenyl,
4) Pyridyl,
5) Chinolyl oder
6) Isochinolyl, wobei das Ringsystem der Reste 3) bis 6) unsubstituiert oder mit einem Rest aus der Gruppe
3.1 Fluoratom,
3.2 Chloratom,
3.3 CF₃,
3.4 Methyl,
3.5 Methoxy oder
3.6 NR²⁵R²⁶
substituiert ist, dabei sind R²⁵ und R²⁶ gleich oder verschieden und haben unabhängig voneinander die Bedeutung
3.6.1 Wasserstoffatom oder
3.6.2 (C₁-C₄)-Alkyl, oder
III) einen Rest der Formel VII 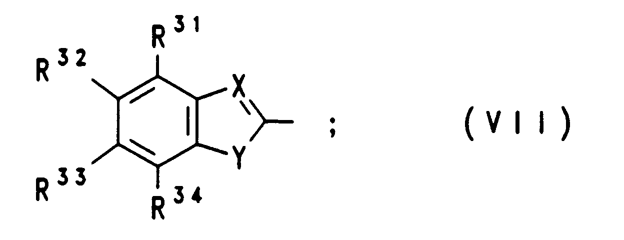 dabei haben R³¹, R³², R³³ oder R³⁴ die nachstehende Bedeutung:
a) Wasserstoffatom,
b) Halogenatom, wie Fluor-, Chlor-, Brom- oder Jodatom,
c) -CN,
d) -NO₂,
e) -N₃,
f) -(C₁-C₆)-Alkyl, geradkettig oder verzweigt oder
g) R³⁵-CₙH₂ₙ-Z-,
dabei ist n die Zahl Null, 1, 2, 3, 4, 5 oder 6, und die Alkylenkette -CₙH₂ₙ- ist geradkettig oder verzweigt, und ein C-Atom kann durch ein O-, S- oder N-Atom ersetzt sein,
R³⁵ ist
1) Wasserstoffatom,
2) (C₃-C₆)-Alkenyl,
3) (C₅-C₈)-Cycloalkyl,
4) (C₅-C₈)-Cycloalkyl, mit einer Hydroxygruppe substituiert, oder
eine Methylengruppe ist durch ein O-, S- oder N-Atom ersetzt, oder
5) Phenyl, unsubstituiert oder substituiert mit 1 bis 3 Resten aus der Gruppe
5.1 Halogenatom, wie Fluor-, Chlor-, Brom- oder Jodatom,
5.2 CF₃,
5.3 CH₃-S(O)ₓ,
dabei ist x die Zahl Null, 1 oder 2,
5.4 R³⁶-Wy,
dabei ist R³⁶ Wasserstoffatom, Methyl oder Ethyl, W ist O-Atom, NH oder NCH₃ und y ist Null oder 1,
5.5 CₘF₂ₘ₊₁,
dabei ist m die Zahl 1, 2 oder 3,
5.6 Pyridyl,
5.7 Chinolyl oder
5.8 Isochinolyl,
Z ist
1) -CO-,
2) -CH₂-,
3) -[CH(OH)]_{q}-,
dabei ist q die Zahl 1, 2 oder 3,
4) -[C(CH₃)(OH)]_{q}-,
dabei ist q die Zahl 1, 2 oder 3,
5) -O-,
6) -NH-,
7) 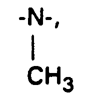
8) -S(O)ₓ-,
dabei ist x Null, 1 oder 2,
9) -SO₂-NH- oder
10) 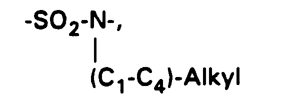
X hat die nachstehende Bedeutung
a) N oder
b) C-R³⁷,
dabei ist R³⁷ Wasserstoffatom, (C₁-C₄)-Alkyl oder (C₂-C₄)-Alkenyl,
Y hat die nachstehende Bedeutung
a) NH,
b) -N-(C₁-C₆)-Alkyl,
c) -N-(C₂-C₄)-Alkenyl oder
d) R³⁵-Cₙ-H₂ₙ-Z-,
dabei ist R³⁵, n und Z wie unter g) definiert,
R⁵, R⁶, R⁷ und R⁸ sind gleich oder verschieden und haben unabhängig voneinander die nachstehende Bedetung
a) Wasserstoffatom,
b) (C₁-C₅)-Alkyl, geradkettig oder verzweigt, oder
c) Phenyl.

2. Verbindung der Formel Ia, Ib oder Ic gemäß Anspruch 1, und/oder ein physiologisch verträgliches Salz der Verbindung der Formel Ia, Ib oder Ic; wobei R die folgende Bedeutung hat:
I) einen Rest der Formel V; dabei hat R¹¹ die nachstehende Bedeutung:
a) Fluoratom,
b) Chloratom,
c) 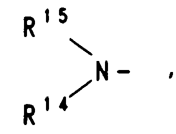 dabei haben R¹⁴ und R¹⁵ die obengenannte Bedeutung,
d) R¹³-S(O)ₙ-, dabei ist n Null, 1 oder 2, oder
e) Phenoxy,
dabei hat R¹² die nachstehende Bedeutung:
a) R¹³-S(O)ₙ, dabei ist n Null, 1 oder 2, oder
b) 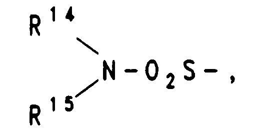 dabei haben R¹⁴ und R¹⁵ die obengenannte Bedeutung, oder
II) einen Rest der Formel VI; dabei haben R²¹, R²² oder R²³ die nachstehende Bedeutung:
a) Wasserstoffatom,
b) Halogenatom wie Fluor-, Chlor- oder Bromatom oder
c) (C₁-C₈)-Alkyl,
wobei einer der Substituenten R²¹, R²² oder R²³ auch bedeuten kann
1) OH,
2) (C₁-C₆)-Alkoxy,
3) R²⁴-CₙH₂ₙ-Oₘ,
dabei ist
m die Zahl Null oder 1,
n ist die Zahl Null, 1, 2 oder 3,
R²⁴ bedeutet
1) CₚF₂ₚ₊₁,
dabei ist p die Zahl 1, sofern
n die Zahl Null oder 1 ist,
2) (C₅-C₇)-Cycloalkyl,
3) Phenyl,
4) Pyridyl,
5) Chinolyl oder
6) Isochinolyl,
wobei das Ringsystem der Reste 3) bis 6) unsubstituiert oder mit einem Rest aus der Gruppe
3.1 Fluoratom,
3.2 Chloratom,
3.3 CF₃,
3.4 CH₃ oder
3.5 Methoxy
substituiert ist, oder
III) einen Rest der Formel VII; dabei haben R³¹, R³², R³³ oder R³⁴ die nachstehende Bedeutung:
a) Wasserstoffatom,
b) Halogenatom, wie Fluor-, Chlor-, Brom- oder Jodatom,
c) (C₁-C₆)-Alkyl, geradkettig oder verzweigt, oder
d) R³⁵-CₙH₂ₙ-Z-,
dabei ist n die Zahl Null, 1 oder 2, und die Alkylenkette -CₙH₂ₙ- ist geradkettig oder verzweigt, und ein C-Atom kann durch ein O-, S- oder N-Atom ersetzt sein,
R³⁵ ist
1) Wasserstoffatom,
2) (C₅-C₈)-Cycloalkyl,
3) (C₅-C₈)-Cycloalkyl, mit einer Hydroxygruppe substituiert, oder
eine Methylengruppe ist durch ein O-, S- oder N-Atom ersetzt, oder
4) Phenyl, unsubstituiert oder substituiert mit 1 bis 3 Resten aus der Gruppe
4.1 Halogenatom, wie Fluor-, Chlor-, Brom- oder Jodatom,
4.2 CF₃,
4.3 CH₃-S(O)ₓ, dabei ist x die Zahl Null, 1 oder 2,
4.4 R³⁶-Wy,
dabei ist R³⁶ Wasserstoffatom, Methyl oder Ethyl, W ist O-Atom, NH oder NCH₃ und y ist Null oder 1,
4.5 CₘF₂ₘ₊₁,
dabei ist m die Zahl 1, 2 oder 3,
4.6 Pyridyl,
4.7 Chinolyl oder
4.8 Isochinolyl,
Z ist
1) -CO-,
2) -CH₂-,
3) -[CH(OH)]_{q}-,
dabei ist q die Zahl 1, 2 oder 3,
4) -[C(CH₃)(OH)]_{q}-,
dabei ist q die Zahl 1, 2 oder 3,
5) -O- oder
6) -S(O)ₓ-,
dabei ist x Null, 1 oder 2,
X hat die nachstehende Bedeutung
a) N oder
b) CH,
Y hat die nachstehende Bedeutung
a) -N-(C₁-C₆)-Alkyl,
b) -N-(C₂-C₄)-Alkenyl oder
c) R³⁵-Cₙ-H₂ₙ-Z-, dabei ist R³⁵, n und Z wie unter d) definiert,
R⁵, R⁶, R⁷ und R⁸ sind gleich oder verschieden und haben unabhängig voneinander die nachstehende Bedetung
a) Wasserstoffatom oder
b) (C₁-C₅)-Alkyl, geradkettig oder verzweigt.

3. Verbindung der Formel Ia, Ib oder Ic gemäß Anspruch 1 oder 2, wobei R¹¹ die folgende Bedeutung hat:
a) 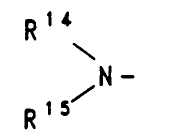 dabei sind R¹⁴ und R¹⁵ gleich oder verschieden und haben unabhängig voneinander die folgende Bedeutung
1) Wasserstoffatom oder
2) (C₁-C₄)-Alkyl, oder
R¹⁴ und R¹⁵ bilden zusammen eine (C₄-C₇)-Alkylkette, oder
b) R¹³-S-,
dabei ist R¹³
a) Phenyl, oder
b) Phenyl, substituiert durch Chlor in para-Position,
dabei hat R¹² die nachstehende Bedeutung:
a) CH₃-SO₂-,
b) H₂N-SO₂-,
c) Phenoxy oder
d) Phenoxy, substituiert durch Chlor in para-Position,
R⁵, R⁶, R⁷ und R⁸ sind gleich oder verschieden und haben unabhängig voneinander die nachstehende Bedeutung
a) Wasserstoffatom oder
b) (C₁-C₄)-Alkyl,
und physiologisch verträgliche Salze der Verbindung der Formel Ia, Ib oder Ic.

4. 2-[(1-Methyl-2-indolylcarbonyl)-(aminoiminomethyl)amino)]-ethan-1,1-bisphosphonsäuretetraethylester, 2-[(1-Methyl-2-indolylcarbonyl)-(aminoiminomethyl)amino)]-ethan-1,1-bisphosphonsäure, 2-[(3-Methylsulfonyl-4-piperidyl-benzoyl)-(aminoiminomethyl)amino)]-ethan-1,1-bisphosphonsäure, 2-[(3-Methylsulfonyl-4-piperidyl-benzoyl)-(aminoiminomethyl)amino)]-ethan-1,1-bisphosphonsäuretetraethylester, 2-[(3,5-Dichlor-benzoyl)-(aminoiminomethyl)amino)]-ethan-1,1-bisphosphon-säuretetraethylester und 2-[(3,5-Dichlor-benzoyl)-(aminoiminomethyl)amino)]-ethan-1,1-bisphosphonsäure.

5. Verfahren zur Herstellung einer Verbindung der tautomeren Formel Ia, Ib oder Ic nach einem oder mehreren der Ansprüche 1 bis 4, dadurch gekennzeichnet, daß man eine Verbindung der Formel IV, 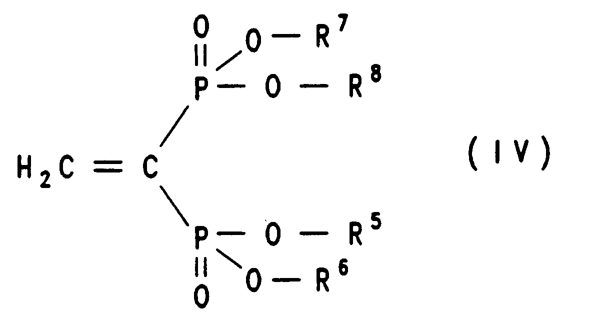 wobei R⁵, R⁶, R⁷ und R⁸ die in Formel Ia gemäß Anspruch 1 genannte Bedeutung haben,
A) mit einer Verbindung der Formel III 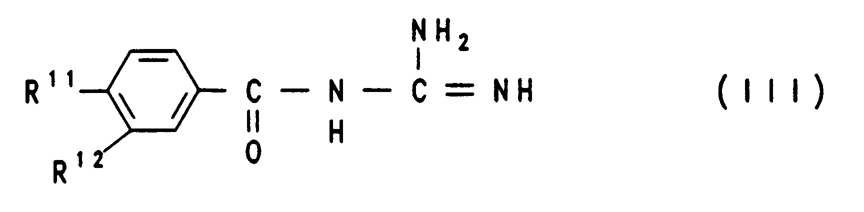 in Gegenwart eines inerten Lösungsmittels zu einer Verbindung der Formel Ia, Ib oder Ic umsetzt, wobei R¹¹ und R¹² die in Formel Ia, Ib oder Ic genannte Bedeutung haben, und gegebenenfalls wird
B) der Bisphosphonsäureester der Verbindung der Formel Ia, Ib oder Ic in die entsprechende Bisphosphonsäure umgesetzt oder
eine Verbindung der Formel IV, wobei R⁵, R⁶, R⁷ und R⁸ die in Formel Ia genannte Bedeutung haben, wird
C) mit einer Verbindung der Formel VIII 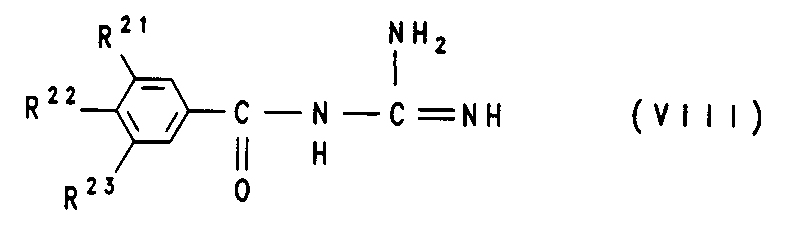 in Gegenwart eines inerten Lösungsmittels zu einer Verbindung der Formel Ia, Ib oder Ic umgesetzt, wobei R²¹, R²² und R²³ die in Formel Ia, Ib oder Ic genannte Bedeutung haben, und gegebenenfalls wird
D) der Bisphosphonsäureester der Verbindung der Formel Ia, Ib oder Ic in die entsprechende Bisphosphonsäure umgesetzt oder
eine Verbindung der Formel IV, wobei R⁵, R⁶, R⁷ und R⁸ die in Formel Ia genannte Bedeutung haben, wird
E) mit einer Verbindung der Formel IX 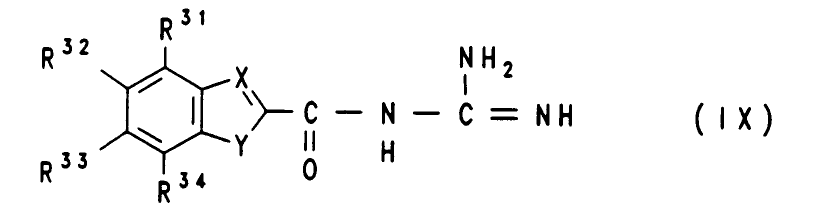 in Gegenwart eines inerten Lösungsmittels zu einer Verbindung der Formel Ia, Ib oder Ic umgesetzt, wobei R³¹, R³², R³³ und R³⁴ die in Formel Ia, Ib oder Ic genannte Bedeutung haben, und gegebenenfalls wird
F) der Bisphosphonsäureester der Verbindung der Formel Ia, Ib oder Ic in die entsprechende Bisphosphonsäure umgesetzt.

6. Arzneimittel, enthaltend eine wirksame Menge von mindestens einer der Verbindungen der tautomeren Formel Ia, Ib oder Ic gemäß Anspruch 1 und/oder mindestens eines der physiologisch verträglichen Salze einer Verbindung der tautomeren Formel Ia, Ib oder Ic, neben pharmazeutisch geeigneten und physiologisch verträglichen Hilfs- und/oder Trägerstoffen, Verdünnungsmitteln und/oder anderen Wirkstoffen.

7. Verfahren zur Herstellung eines Arzneimittels nach Anspruch 6, dadurch gekennzeichnet, daß man mindestens eine Verbindung der tautomeren Formel Ia, Ib oder Ic und/oder mindestens eines der physiologisch verträglichen Salze der Verbindung der tautomeren Formel Ia, Ib oder Ic, gegebenenfalls mit Hilfs-und/oder Trägerstoffen, Verdünnungsmitteln und/oder anderen Wirkstoffen, in eine geeignete Darreichungsform gebracht wird.

8. Verwendung einer Verbindung der Formel Ia, Ib oder Ic nach einem oder mehreren der Ansprüche 1 bis 4 und/oder wie erhalten nach dem Verfahren nach Anspruch 5 zur Herstellung eines Arzneimittels zur Prophylaxe oder Behandlung von degenerativen Erkrankungen des Knochensystems.

9. Verwendung nach Anspruch 8 zur Herstellung eines Arzneimittels zur Behandlung von Erkrankungen mit erhöhter Knochenresorption, insbesondere Paget-disease, metastasischer Osteokarcenoma, Hypercalcemia oder Osteoporose.

## Claims

1. A compound of the tautomeric formula Ia, Ib or Ic 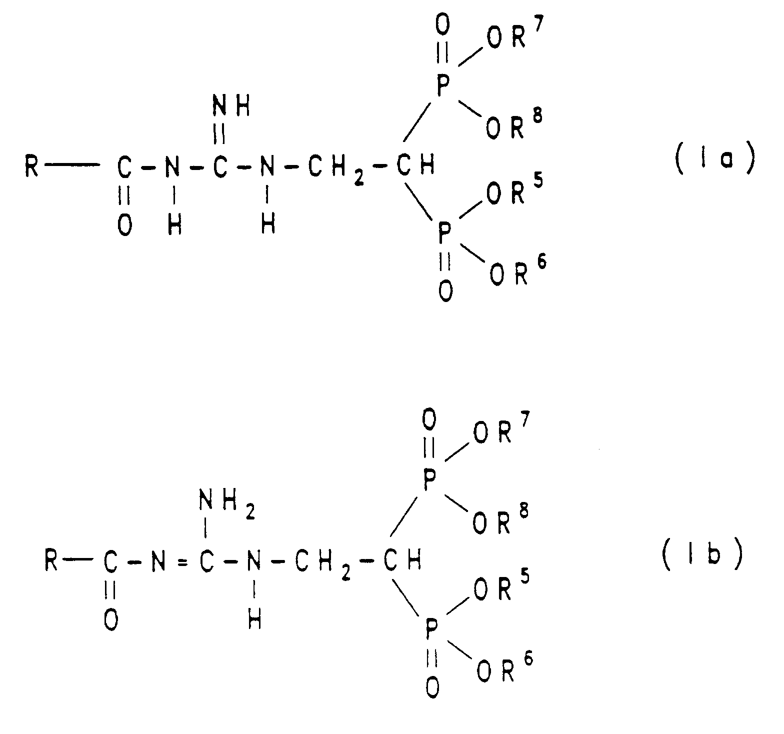 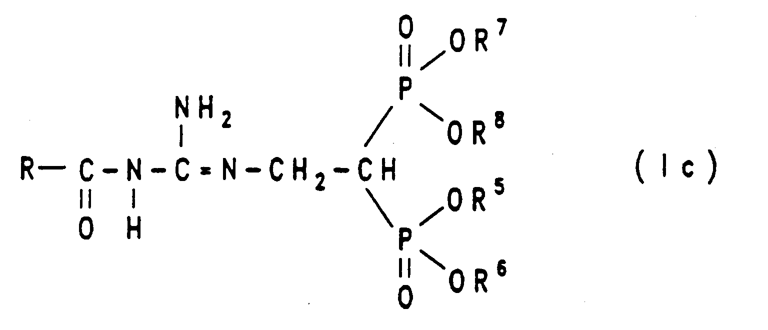 and/or a physiologically tolerated salt of the compound of the formula Ia, Ib or Ic; where R has the following meaning:
I) a radical of the formula V 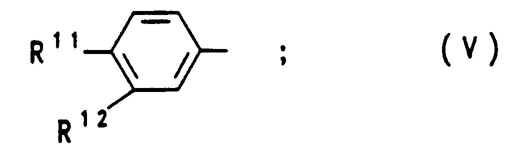 where R¹¹ or R¹² has the following meaning:
a) R¹³-S(O)ₙ- or 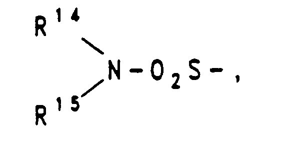
b) where R¹³ is
1) (C₁-C₆)-alkyl,
2) (C₅-C₇)-cycloalkyl,
3) cyclopentylmethyl,
4) cyclohexylmethyl,
5) phenyl,
6) phenyl substituted once to three times by
6.1 fluorine atom,
6.2 chlorine atom,
6.3 methyl or
6.4 methoxy,
where n is the integer zero, 1 or 2, where R¹⁴ and R¹⁵ are identical or different and have, independently of one another, the following meaning:
1) hydrogen atom,
2) (C₁-C₆)-alkyl,
3) phenyl,
4) phenyl substituted once or twice by
4.1 fluorine atom,
4.2 chlorine atom,
4.3 methyl or
4.4 methoxy,
5) -(CH₂)ₘ-phenyl where m is an integer from 1 to 4, or
6) -(CH₂)ₘ-phenyl where m is an integer from 1 to 4 and the phenyl radical is substituted once or twice by the radicals indicated in 4.1 to 4.4,
7) R¹⁴ and R¹⁵ together form a straight-chain or branched chain of 4 to 7 carbon atoms,
the chain can additionally be interrupted by
7.1 O,
7.2 S or
7.3 NR¹⁰,
where
R¹⁰ is
1) hydrogen atom or
2) methyl, or
8) R¹⁴ and R¹⁵ form together with the nitrogen atom to which they are bonded a
8.1 dihydroindole,
8.2 tetrahydroquinoline or
8.3 tetrahydroisoquinoline ring,
and the other substituent R¹¹ or R¹² in each case means
a) hydrogen atom,
b) halogen atom, such as fluorine, chlorine, bromine or iodine atom,
c) (C₁-C₄)-alkyl,
d) (C₁-C₄)-alkoxy,
e) phenoxy,
f) phenoxy substituted once to three times by
1) fluorine or chlorine atom,
2) methyl or
3) methoxy,
g) R¹³-S(O)ₙ, where n is the integer zero, 1 or 2 and R¹³ has the abovementioned meaning, or h) 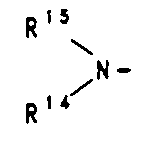 where R¹⁴ and R¹⁵ have the above-mentioned meaning, or
II) a radical of the formula VI 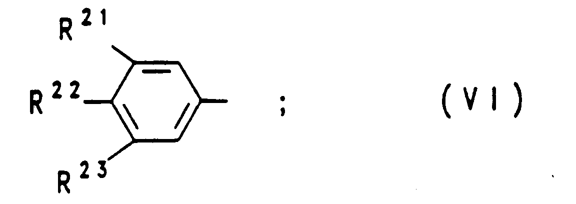 where R²¹, R²² or R²³ has the following meaning:
a) hydrogen atom,
b) halogen atom, such as fluorine, chlorine, bromine or iodine atom, or
c) (C₁-C₁₂)-alkyl,
where one of the substituents R²¹, R²² or R²³ can also mean
1) N₃,
2) CN,
3) OH,
4) (C₁-C₁₀)-alkoxy,
5) R²⁴-CₙH₂ₙ-Oₘ,
where
m is the number zero or 1,
n is the number zero, 1, 2 or 3,
R²⁴ is
1) CₚF₂ₚ₊₁ where
p is the number 1, 2 or 3 as long as
n is the number zero or 1,
2) (C₃-C₁₂)-cycloalkyl,
3) phenyl,
4) pyridyl,
5) quinolyl or
6) isoquinolyl,
where the ring system in the radicals 3) to 6) is unsubstituted or substituted by a radical from the group
3.1 fluorine atom,
3.2 chlorine atom,
3.3 CF₃,
3.4 methyl,
3.5 methoxy or
3.6 NR²⁵R²⁶,
where R²⁵ and R²⁶ are identical or different and have, independently of one another, the meaning
3.6.1 hydrogen atom or
3.6.2 (C₁-C₄)-alkyl, or
III) a radical of the formula VII 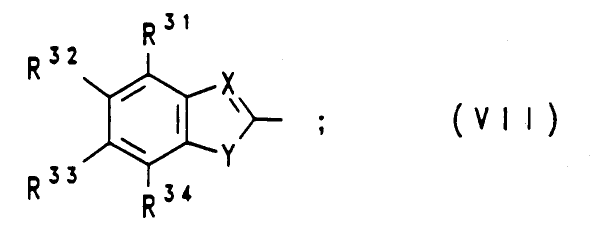 where R³¹, R³², R³³ or R³⁴ has the following meaning:
a) hydrogen atom,
b) halogen atom, such as fluorine, chlorine, bromine or iodine atom,
c) -CN,
d) -NO₂,
e) -N₃,
f) -(C₁-C₆)-alkyl, straight-chain or branched or
g) R³⁵-CₙH₂ₙ-Z-,
where n is the number zero, 1, 2, 3, 4, 5 or 6, and the alkylene chain -CₙH₂ₙ- is straight-chain or branched, and one carbon atom can be replaced by an oxygen, sulfur or nitrogen atom,
R³⁵ is
1) hydrogen atom,
2) (C₃-C₆)-alkenyl,
3) (C₅-C₈)-cycloalkyl,
4) (C₅-C₈)-cycloalkyl, substituted by a hydroxyl group, or
one methylene group is replaced by an oxygen, sulfur or nitrogen atom, or
5) phenyl, unsubstituted or substituted by 1 to 3 radicals from the group
5.1 halogen atom, such as fluorine, chlorine, bromine or iodine atom,
5.2 CF₃,
5.3 CH₃-S(O)ₓ,
where x is the number zero, 1 or 2,
5.4 R³⁶-W_{y},
where R³⁶ is hydrogen atom, methyl or ethyl, W is oxygen atom, NH or NCH₃, and y is zero or 1,
5.5 CₘF₂ₘ₊₁,
where m is the number 1, 2 or 3,
5.6 pyridyl,
5.7 quinolyl or
5.8 isoquinolyl,
Z is
1) -CO-,
2) -CH₂-,
3) -[CH(OH)]_{q}-,
where q is the number 1, 2 or 3,
4) -[C(CH₃)(OH)]_{q}-,
where q is the number 1, 2 or 3,
5) -O-,
6) -NH-,
7) 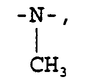
8) -S(O)ₓ-,
where x is zero, 1 or 2,
9) -SO₂-NH- or
10) 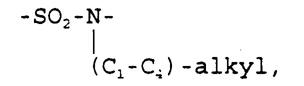
X has the following meaning
a) N or
b) C-R³⁷,
where R³⁷ is hydrogen atom, (C₁-C₄)-alkyl or (C₂-C₄)-alkenyl,
Y has the following meaning
a) NH,
b) -N-(C₁-C₆)-alkyl,
c) -N-(C₂-C₄)-alkenyl or
d) R³⁵-CₙH₂ₙ-Z-,
where R³⁵, n and Z are defined as under g),
R⁵, R⁶, R⁷ and R⁸ are identical or different and have, independently of one another, the following meaning
a) hydrogen atom,
b) (C₁-C₅)-alkyl, straight-chain or branched, or
c) phenyl.

2. A compound of the formula Ia, Ib or Ic as claimed in claim 1, and/or a physiologically tolerated salt of the compound of the formula Ia, Ib or Ic, where R has the following meaning:
I) a radical of the formula V, where R¹¹ has the following meaning:
a) fluorine atom,
b) chlorine atom,
c) 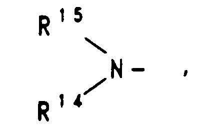 where R¹⁴ and R¹⁵ have the abovementioned meaning,
d) R¹³-S(O)ₙ-, where n is zero, 1 or 2, or
e) phenoxy,
where R¹² has the following meaning:
a) R¹³-S(O)ₙ-, where n is zero, 1 or 2, or
b) 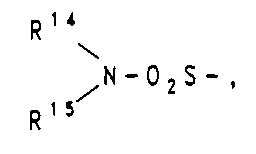 where R¹⁴ and R¹⁵ have the abovementioned meaning, or
II) a radical of the formula VI, where R²¹, R²² or R²³ has the following meaning:
a) hydrogen atom,
b) halogen atom such as fluorine, chlorine or bromine atom, or
c) (C₁-C₈)-alkyl,
where one of the substituents R²¹, R²² or R²³ can also mean
1) OH,
2) (C₁-C₆)-alkoxy,
3) R²⁴-CₙH₂ₙ-Oₘ,
where
m is the number zero or 1,
n is the number zero, 1, 2 or 3,
R²⁴ means
1) CₚF₂ₚ₊₁,
where
p is the number 1 as long as
n is the number zero or 1,
2) (C₅-C₇)-cycloalkyl,
3) phenyl,
4) pyridyl,
5) quinolyl or
6) isoquinolyl,
where the ring system in the radicals 3) to 6) is unsubstituted or substituted by a radical from the group
3.1 fluorine atom,
3.2 chlorine atom,
3.3 CF₃,
3.4 CH₃ or
3.5 methoxy, or
III) a radical of the formula VII, where R³¹, R³², R³³ or R³⁴ has the following meaning:
a) hydrogen atom,
b) halogen atom, such as fluorine, chlorine, bromine or iodine atom,
c) (C₁-C₆)-alkyl, straight-chain or branched, or
d) R³⁵-CₙH₂ₙ-Z-,
where n is the number zero, 1 or 2, and the alkylene chain -CₙH₂ₙ- is straight-chain or branched, and one carbon atom can be replaced by an oxygen, sulfur or nitrogen atom,
R³⁵ is
1) hydrogen atom,
2) (C₅-C₈)-cycloalkyl,
3) (C₅-C₈)-cycloalkyl,
substituted by a hydroxyl group, or
one methylene group is replaced by an oxygen, sulfur or nitrogen atom, or
4) phenyl, unsubstituted or substituted by 1 to 3 radicals from the group
4.1 halogen atom, such as fluorine, chlorine, bromine or iodine atom,
4.2 CF₃,
4.3 CH₃-S(O)ₓ,
where x is the number zero, 1 or 2,
4.4 R³⁶-W_{y},
where R³⁶ is hydrogen atom, methyl or ethyl, W is oxygen atom, NH or NCH₃, and y is zero or 1,
4.5 CₘF₂ₘ₋₁,
where m is the number 1, 2 or 3,
4.6 pyridyl,
4.7 quinolyl or
4.8 isoquinolyl,
Z is
1) -CO-,
2) -CH₂-,
3) -[CH(OH)]_{q}-,
where q is the number 1, 2 or 3,
4) -[C(CH₃)(OH)]_{q}-,
where q is the number 1, 2 or 3,
5) -O- or
6) -S(O)ₓ-,
where x is zero, 1 or 2,
X has the following meaning
a) N or
b) CH,
Y has the following meaning
a) -N-(C₁-C₆)-alkyl,
b) -N-(C₂-C₄)-alkenyl or
c) R³⁵-CₙH₂ₙ-Z-,
where R³⁵, n and Z are defined as under d),
R⁵, R⁶, R⁷ and R⁸ are identical or different and have, independently of one another, the following meaning
a) hydrogen atom or
b) (C₁-C₅)-alkyl, straight-chain or branched.

3. A compound of the formula Ia, Ib or Ic as claimed in claim 1 or 2, where R¹¹ has the following meaning:
a) 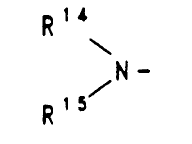 where R¹⁴ and R¹⁵ are identical or different and have, independently of one another, the following meaning
1) hydrogen atom or
2) (C₁-C₄)-alkyl, or
R¹⁴ and R¹⁵ together form a (C₄-C₇)-alkyl chain, or
b) R¹³-S-,
where R¹³ is
a) phenyl, or
b) phenyl, substituted by chlorine in the para position,
where R¹² has the following meaning:
a) CH₃-SO₂-,
b) H₂N-SO₂-,
c) phenoxy or
d) phenoxy, substituted by chlorine in the para position,
R⁵, R⁶, R⁷ and R⁸ are identical or different and have, independently of one another, the following meaning
a) hydrogen atom or
b) (C₁-C₄)-alkyl,
and physiologically tolerated salts of the compound of the formula Ia, Ib or Ic.

4. Tetraethyl 2-[(1-methyl-2-indolylcarbonyl)-(amino-iminomethyl)amino]ethane-1,1-bisphosphonate, 2-[(1-methyl-2-indolylcarbonyl)-(aminoiminomethyl)-amino]ethane-1,1-bisophosphonic acid, 2-[(3-methylsulfonyl-4-piperidylbenzoyl)-(aminoiminomethyl)-amino]ethane-1,1-bisphosphonic acid, tetraethyl 2-[(3-methylsulfonyl-4-piperidylbenzoyl)-(aminoiminomethyl)amino]ethane-1,1-bisphosphonate, tetraethyl 2-[(3,5-dichlorobenzoyl)-(aminoiminomethyl)amino]ethane-1,1-bisphosphonate and 2-[(3,5-dichlorobenzoyl)-(aminoiminomethyl)amino]-ethane-1,1-bisphosphonic acid.

5. A process for the preparation of a compound of the tautomeric formula Ia, Ib or Ic as claimed in one or more of claims 1 to 4, which comprises a compound of the formula IV 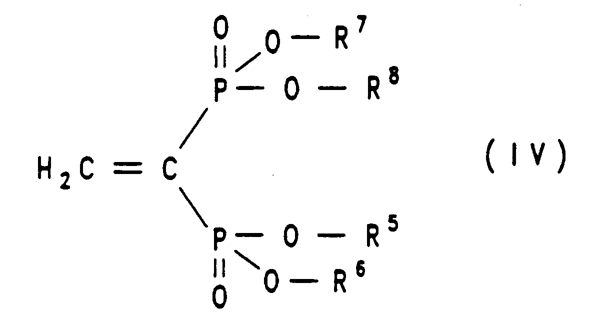 where R⁵, R⁶, R⁷ and R⁸ have the meaning mentioned in formula Ia as defined in claim 1, being
A) reacted with a compound of the formula III 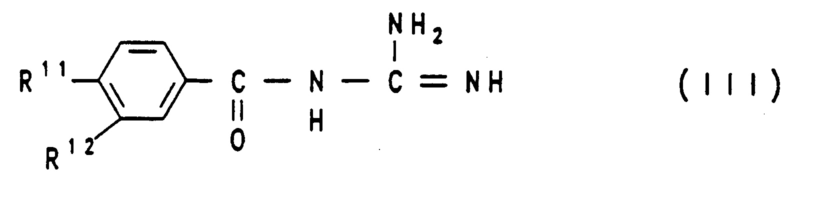 in the presence of an inert solvent to give a compound of the formula Ia, Ib or Ic, where R¹¹ and R¹² have the meaning mentioned in formula Ia, Ib or Ic, and, where appropriate,
B) the bisphosphonic ester of the compound of the formula Ia, Ib or Ic being converted into the corresponding bisphosphonic acid, or
a compound of the formula IV, where R⁵, R⁶, R⁷ and R⁸ have the meaning mentioned in formula Ia, being
C) reacted with a compound of the formula VIII 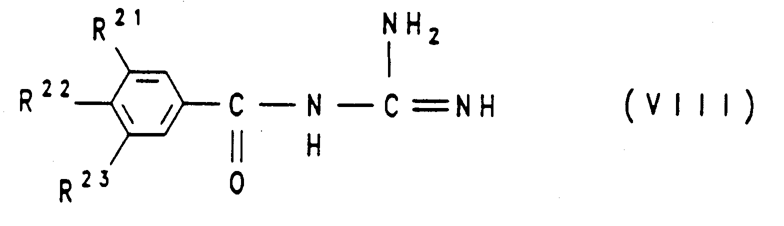 in the presence of an inert solvent to give a compound of the formula Ia, Ib or Ic, where R²¹, R²² and R²³ have the meaning mentioned in formula Ia, Ib or Ic, and, where appropriate,
D) the bisphosphonic ester of the compound of the formula Ia, Ib or Ic being converted into the corresponding bisphosphonic acid, or
a compound of the formula IV, where R⁵, R⁶, R⁷ and R⁸ have the meaning mentioned in formula Ia, being
E) reacted with a compound of the formula IX 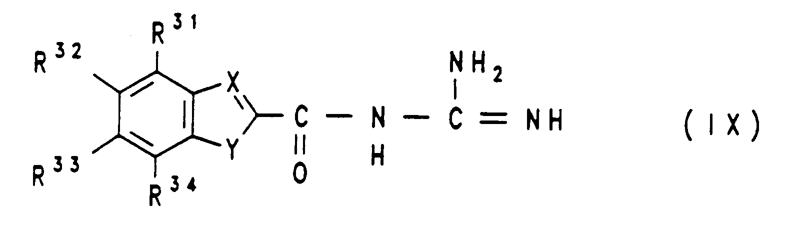 in the presence of an inert solvent to give a compound of the formula Ia, Ib or Ic, where R³¹, R³², R³³ and R³⁴ have the meaning mentioned in formula Ia, Ib or Ic, and, where appropriate,
F) the bisphosphonic ester of the compound of the formula Ia, Ib or Ic being converted into the corresponding bisphosphonic acid.

6. A pharmaceutical containing an effective amount of at least one of the compounds of the tautomeric formula Ia, Ib or Ic as claimed in claim 1 and/or at least one of the physiologically tolerated salts of a compound of the tautomeric formula Ia, Ib or Ic, in addition to pharmaceutically acceptable and physiologically tolerated ancillary substances and/or excipients, diluents and/or other active substances.

7. A process for the production of a pharmaceutical as claimed in claim 6, which comprises converting at least one compound of the tautomeric formula Ia, Ib or Ic and/or at least one of the physiologically tolerated salts of the compound of the tautomeric formula Ia, Ib or Ic, where appropriate with ancillary substances and/or excipients, diluents and/or other active substances, into a suitable dosage form.

8. The use of a compound of the formula Ia, Ib or Ic as claimed in one or more of claims 1 to 4 and/or as obtained by the process as claimed in claim 5 for the production of a pharmaceutical for the prophylaxis or treatment of degenerative disorders of the bone system.

9. The use as claimed in claim 8 for the production of a pharmaceutical for the treatment of disorders with increased bone resorption, in particular Paget's disease, metastatic osteocarcinoma, hypercalcemia or osteoporosis.

## Revendications

1. Composé de formule tautomère Ia, Ib ou Ic 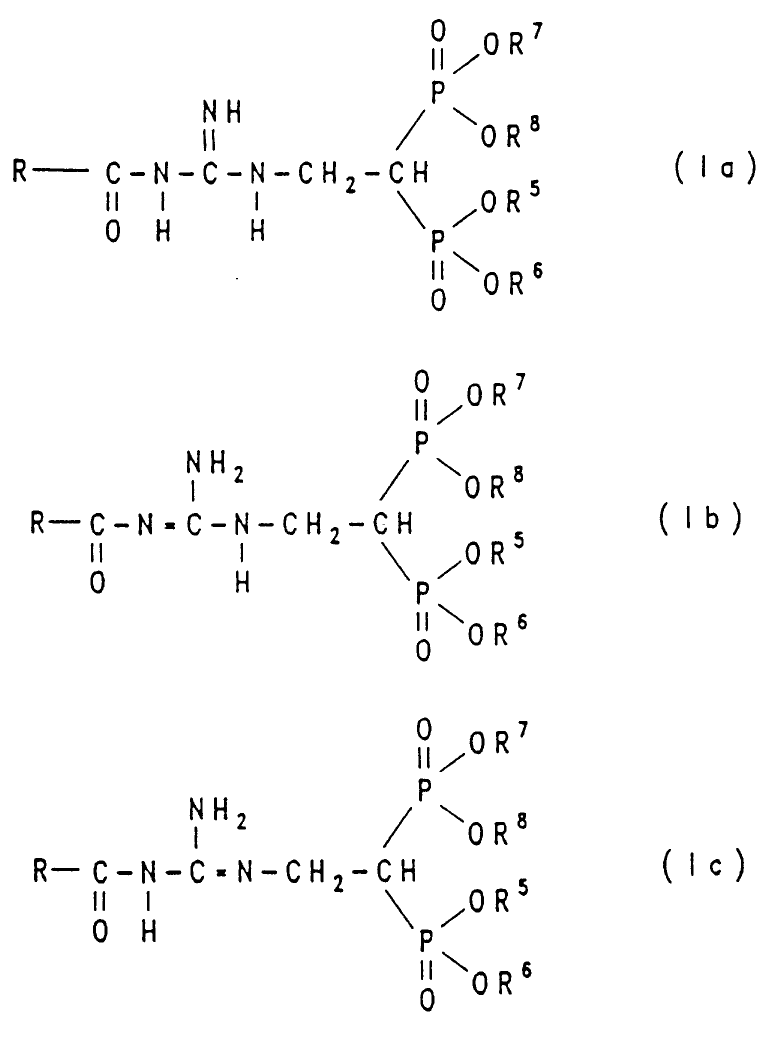 et/ou sel physiologiquement acceptable du composé de formule Ia, Ib ou Ic.
R ayant la signification suivante :
I) un radical de formule V 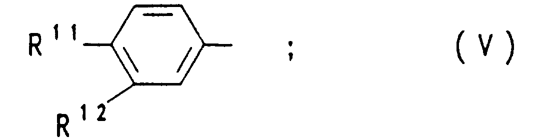 dans laquelle R¹¹ ou R¹² ont les significations sui-suivantes:
a) R¹³-S(O)ₙ ou
b) 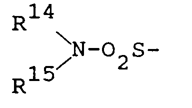 R¹³ représentant
1) un groupe alkyle en C₁-C₆,
2) un groupe cycloalkyle en C₅-C₇,
3) le groupe cyclopentylméthyle,
4) le groupe cyclohexylméthyle,
5) le groupe phényle,
6) un groupe phényle une à trois fois substitué par
6.1 l'atome de fluor,
6.2 l'atome de chlore,
6.3 le groupe méthyle ou
6.4 le groupe méthoxy,
n étant le nombre entier zéro, 1 ou 2,
R¹⁴ et R¹⁵ étant identiques ou différents et ayant, indépendamment l'un de l'autre, les significations suivantes:
1) un atome d'hydrogène,
2) un groupe alkyle en C₁-C₆,
3) le groupe phényle,
4) un groupe phényle une ou deux fois substitué par
4.1 l'atome de fluor,
4.2 l'atome de chlore,
4.3 le groupe méthyle ou
4.4 le groupe méthoxy,
5) un groupe -(CH₂)ₘ-phényle, m étant un nombre entier allant de 1 à 4, ou
6) un groupe -(CH₂)ₘ-phényle, m étant un nombre entier allant de 1 à 4 et le radical phényle étant une ou deux fois substitué par les radicaux indiqués en 4.1 à 4.4,
7) R¹⁴ et R¹⁵ forment ensemble une chaîne droite ou ramifiée ayant de 4 à 7 atomes de carbone, la chaîne pouvant en outre être interrompue par
7.1 O,
7.2 S ou
7.3 NR¹⁰,
R¹⁰ représentant
1) un atome d'hydrogène ou
2) le groupe méthyle, ou
8) R¹⁴ et R¹⁵ forment ensemble, avec l'atome d'azote auquel il sont liés, un cycle
8.1 dihydro-indole,
8.2 tétrahydroquinoléine ou
8.3 tétrahydro-isoquinoléine,
et l'autre substituant R¹¹ ou R¹² représente
a) un atome d'hydrogène,
b) un atome d'halogène, tel qu'un atome de fluor, chlore, brome ou iode,
c) un groupe alkyle en C₁-C₄,
d) un groupe alcoxy en C₁-C₄,
e) le groupe phénoxy,
f) un groupe phénoxy une à trois fois substitué par
1) l'atome de fluor ou de chlore,
2) le groupe méthyle ou
3) le groupe méthoxy,
g) un groupe R¹³-S(O)ₙ, n étant le nombre entier zéro, 1 ou 2 et R¹³ ayant la signification donnée plus haut, ou
h) 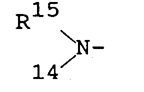 R¹⁴ et R¹⁵ ayant les significations données plus haut, ou
II) un radical de formule VI 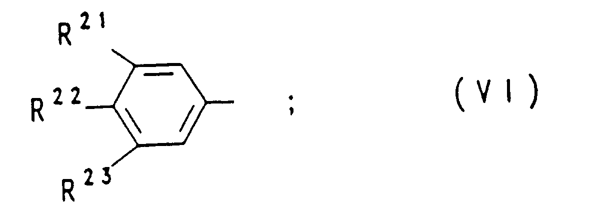 dans laquelle R²¹, R²² ou R²³ ont les significations suivantes:
a) un atome d'hydrogène,
b) un atome d'halogène, tel qu'un atome de fluor, chlore, brome ou iode, ou
c) un groupe alkyle en C₁-C₁₂,
l'un des substituants R²¹, R²² ou R²³ pouvant également représenter
1) N₃,
2) CN,
3) OH,
4) un groupe alcoxy en C₁-C₁₀,
5) un groupe R²⁴-CₙH₂ₙ-Oₘ,
m étant le nombre zéro ou 1,
n étant le nombre zéro, 1, 2 ou 3,
R²⁴ étant
1) CₚF_{2p+1,}
p étant le nombre 1, 2 ou 3,
n étant le nombre zéro ou 1,
2) un groupe cycloalkyle en C₃-C₁₂,
3) le groupe phényle,
4) le groupe pyridyle,
5) le groupe quinolyle ou
6) le groupe isoquinolyle,
le système cyclique des radicaux 3) à 6) étant non substitué ou portant un substituant choisi parmi
3.1 un atome de fluor,
3.2 un atome de chlore,
3.3 CF₃,
3.4 le groupe méthyle,
3.5 le groupe méthoxy ou
3.6 NR²⁵R²⁶,
R²⁵ et R²⁶ étant identiques ou différents et ayant, indépendamment l'un de l'autre, les significations suivantes:
3.6.1 un atome d'hydrogène ou
3.6.2 un groupe alkyle en C₁-C₄, ou
III) un radical de formule VII 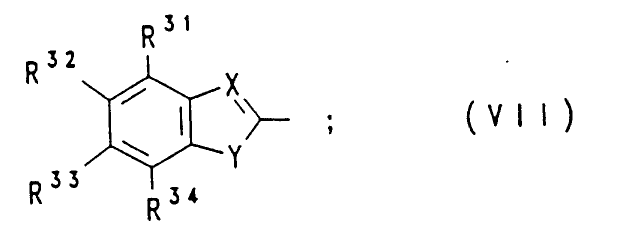 dans laquelle R³¹, R³², R³³ ou R³⁴ ont les significations suivantes:
a) un atome d'hydrogène,
b) un atome d'halogène, tel qu'un atome de fluor, chlore, brome ou iode,
c) -CN,
d) -NO₂,
e) -N₃,
f) un groupe alkyle en C₁-C₆ linéaire ou ramifié, ou
g) un groupe R³⁵-CₙH₂ₙ-Z-
dans lequel n est le nombre zéro, 1, 2, 3, 4, 5 ou 6, et la chaîne alkylène -CₙH₂ₙ- est linéaire ou ramifiée, et un atome de carbone peut être remplacé par un atome d'oxygène, de soufre ou d'azote,
R³⁵ est
1) un atome d'hydrogène,
2) un groupe alcényle en C₃-C₆,
3) un groupe cycloalkyle en C₅-C₈,
4) un groupe cycloalkyle en C₅-C₈ substitué par un groupe hydroxyle, ou
un groupe méthylène est remplacé par un atome d'oxygène, de soufre ou d'azote, ou
5) un groupe phényle non substitué ou portant 1 à 3 substituants choisis dans l'ensemble constitué par
5.1 un atome d'halogène, tel qu'un atome de fluor, chlore, brome ou iode,
5.2 CF₃,
5.3 CH₃-S(O)ₓ,
x étant le nombre zéro, 1 ou 2,
5.4 R³⁶-Wy,
R³⁶ étant un atome d'hydrogène, le groupe méthyle ou éthyle, W étant un atome d'oxygène, NH ou NCH₃ et y étant zéro ou 1,
5.5 CₘF₂ₘ₊₁,
m étant le nombre 1, 2 ou 3,
5.6 le groupe pyridyle,
5.7 le groupe quinolyle ou
5.8 le groupe isoquinolyle,
Z est
1) -CO-,
2) -CH₂-,
3) -[CH(OH)]_{q}-,
q étant le nombre 1, 2 ou 3,
4) -[C(CH₃)(OH)]_{q}-,
q étant le nombre 1, 2 ou 3,
5) -O-,
6) -NH-,
7) 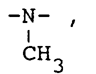
8) -S(O)ₓ-,
x étant zéro, 1 ou 2,
9) -SO₂-NH- ou
10) 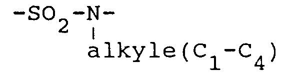
X a la signification suivante
a) N,
b) C-R³⁷,
R³⁷ étant un atome d'hydrogène ou un groupe alkyle en C₁-C₄ ou alcényle en C₂-C₄,
Y a la signification suivante
a) NH,
b) un groupe -N-alkyle(C₁-C₆),
c) un groupe -N-alcényle(C₂-C₄) ou
d) un groupe R³⁵-Cₙ-H₂ₙ-Z-
dans lequel R³⁵, n et Z sont tels que définis en g),
R⁵, R⁶, R⁷ et R⁸ sont identiques ou différents et ont, indépendamment les uns des autres, les significations suivantes:
a) un atome d'hydrogène,
b) un groupe alkyle en C₁-C₅ linéaire ou ramifié, ou
c) le groupe phényle.

2. Composé de formule Ia, Ib ou Ic selon la revendication 1, et/ou sel physiologiquement acceptable du composé de formule Ia, Ib ou Ic, R ayant la signification suivante:
I) un radical de formule V, dans laquelle R¹¹ a la signification suivante:
a) un atome de fluor,
b) un atome de chlore,
c) 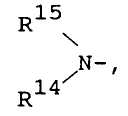 R¹⁴ et R¹⁵ ayant les significations données plus haut,
d) R¹³-S(O)ₙ-, n étant zéro, 1 ou 2, ou
e) le groupe phénoxy,
R¹² a la signification suivante:
a) R¹³-S(O)ₙ-, n étant zéro, 1 ou 2, ou
b) 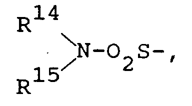 R¹⁴ et R¹⁵ ayant les significations données plus haut, ou
II) un radical de formule VI, dans laquelle R²¹, R²² ou R²³ ont les significations suivantes:
a) un atome d'hydrogène,
b) un atome d'halogène, tel qu'un atome de fluor, chlore ou brome, ou
c) un groupe alkyle en C₁-C₈,
l'un des substituants R²¹, R²² ou R²³ pouvant également représenter
1) OH,
2) un groupe alcoxy en C₁-C₆,
3) un groupe R²⁴-CₙH₂ₙ-Oₘ,
m étant le nombre zéro ou 1,
n étant le nombre zéro, 1, 2 ou 3,
R²⁴ représentant
1) CₚF₂ₚ₊₁,
p étant le nombre 1, dans la mesure où
n est le nombre zéro ou 1,
2) un groupe cycloalkyle en C₅-C₇,
3) le groupe phényle,
4) le groupe pyridyle,
5) le groupe quinolyle ou
6) le groupe isoquinolyle,
le système cyclique des radicaux 3) à 6) étant non substitué ou portant un substituant choisi parmi
3.1 un atome de fluor,
3.2 un atome de chlore,
3.3 CF₃,
3.4 CH₃,
3.5 le groupe méthoxy, ou
III) un radical de formule VII, dans laquelle R³¹, R³², R³³ ou R³⁴ ont les significations suivantes:
a) un atome d'hydrogène,
b) un atome d'halogène, tel qu'un atome de fluor, chlore, brome ou iode,
c) un groupe alkyle en C₁-C₆ linéaire ou ramifié, ou
d) un groupe R³⁵-CₙH₂ₙ-Z-
dans lequel n est le nombre zéro, 1 ou 2, et la chaîne alkylène -CₙH₂ₙ- est linéaire ou ramifiée, et un atome de carbone peut être remplacé par un atome d'oxygène, de soufre ou d'azote,
R³⁵ est
1) un atome d'hydrogène
3) un groupe cycloalkyle en C₅-C₈,
3) un groupe cycloalkyle en C₅-C₈ substitué par un groupe hydroxyle, ou
un groupe méthylène est remplacé par un atome d'oxygène, de soufre ou d'azote, ou
4) un groupe phényle non substitué ou portant 1 à 3 substituants choisis dans l'ensemble constitué par
4.1 un atome d'halogène, tel qu'un atome de fluor, chlore, brome ou iode,
4.2 CF₃,
4.3 CH₃-S(O)ₓ,
x étant le nombre zéro, 1 ou 2,
5.4 R³⁶-Wy,
R³⁶ étant un atome d'hydrogène, le groupe méthyle ou éthyle, W étant un atome d'oxygène, NH ou NCH₃ et y étant zéro ou 1,
4.5 CₘF₂ₘ₊₁,
m étant le nombre 1, 2 ou 3,
4.6 le groupe pyridyle,
4.7 le groupe quinolyle ou
4.8 le groupe isoquinolyle,
Z est
1) -CO-,
2) -CH₂-,
3) -[CH(OH)]_{q}-,
q étant le nombre 1, 2 ou 3,
4) -[C(CH₃)(OH)]_{q}-,
q étant le nombre 1, 2 ou 3,
5) -O- ou
6 -S(O)ₓ-,
x étant zéro, 1 ou 2,
X a la signification suivante
a) N ou
b) CH,
Y a la signification suivante
a) un groupe -N-alkyle(C₁-C₆),
c) un groupe -N-alcényle(C₂-C₄) ou
d) un groupe R³⁵-Cₙ-H₂ₙ-Z-
dans lequel R³⁵, n et Z sont tels que définis en d),
R⁵, R⁶, R⁷ et R⁸ sont identiques ou différents et ont, indépendamment les uns des autres, les significations suivantes:
a) un atome d'hydrogène ou
b) un groupe alkyle en C₁-C₅ linéaire ou ramifié.

3. Composé de formule Ia, Ib ou Ic selon la revendication 1 ou 2, dans lequel R¹¹ a la signification suivante:
a) 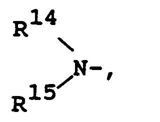 R¹⁴ et R¹⁵ étant identiques ou différents et ayant, indépendamment l'un de l'autre, la signification suivante:
1) un atome d'hydrogène ou
2) un groupe alkyle en C₁-C₄, ou
R¹⁴ et R¹⁵ forment ensemble une chaîne alkyle en C₄-C₇, ou
b) R¹³-S-,
R¹³ représentant
a) le groupe phényle ou,
b) un groupe phényle substitué par un atome de chlore en position para,
en outre R¹² a la signification suivante:
a) CH₃-SO₂-,
b) H₂N-SO₂-,
c) le groupe phénoxy ou
d) un groupe phénoxy substitué par un atome de chlore en position para,
R⁵, R⁶, R⁷ et R⁸ sont identiques ou différents et ont, indépendamment les uns des autres, la signification suivante
a) un atome d'hydrogène ou
b) un groupe alkyle en C₁-C₄,
et sels physiologiquement acceptables du composé de formule Ia, Ib ou Ic.

4. 2-[(1-méthyl-2-indolylcarbonyl)-(amino-iminométhylamino)]éthane-1,1-diphosphonate de tétraéthyle, acide 2-[(1-méthyl-2-indolylcarbonyl)-(amino-iminométhylamino)]éthane-1,1-diphosphonique, acide 2-[(3-méthylsulfonyl-4-pipéridylbenzoyl)-(amino-iminométhylamino)]-éthane-1,1-diphosphonique, 2-[(3-méthylsulfonyl-4-pipéridylbenzoyl)-(amino-iminométhylamino)]éthane-1,1-diphosphonate de tétraéthyle, 2-[(3,5-dichlorobenzoyl)-(amino-iminométhylamino)]éthane-1,1-diphosphonate de tétraéthyle et acide 2-[(3,5-dichlorobenzoyl)-(amino-iminométhylamino)]éthane-1,1-diphosphonique.

5. Procédé pour la préparation d'un composé de formule tautomère Ia, Ib ou Ic selon une ou plusieurs des revendications 1 à 4, caractérisé en ce que l'on fait réagir un composé de formule IV, 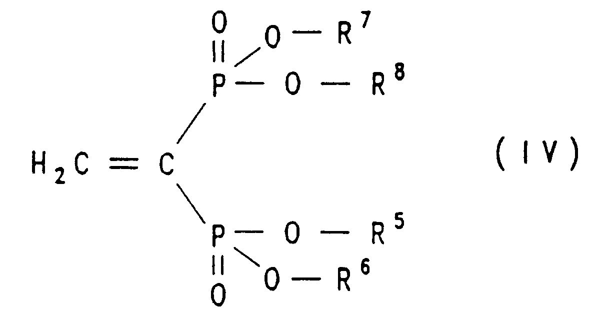 dans laquelle R⁵, R⁶, R⁷ et R⁸ ont les significations données dans la formule Ia selon la revendication 1,
A) avec un composé de formule III 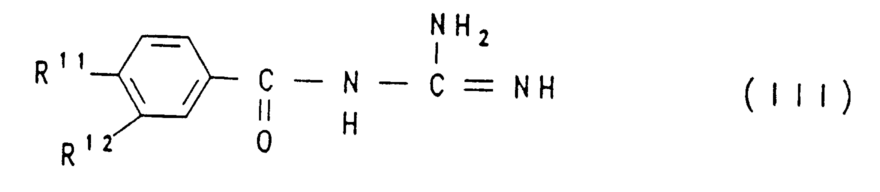 en présence d'un solvant inerte, pour obtenir un composé de formule Ia, Ib ou Ic, R¹¹ et R¹² ayant les significations données dans la formule Ia, Ib ou Ic, et éventuellement
B) on convertit l'ester d'acide diphosphonique du composé de formule Ia, Ib ou Ic en l'acide diphosphonique correspondant, ou
un composé de formule IV, dans laquelle R⁵, R⁶, R⁷ et R⁸ ont les significations données dans la formule Ia,
C) avec un composé de formule VIII 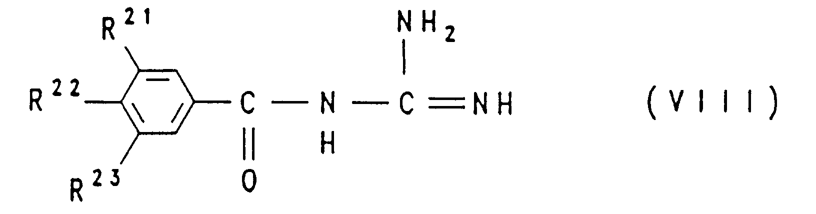 en présence d'un solvant inerte, pour obtenir un composé de formule Ia, Ib ou Ic, R²¹, R²² et R²³ ayant les significations données dans la formule Ia, Ib ou Ic, et éventuellement
D) on convertit l'ester d'acide diphosphonique du composé de formule Ia, Ib ou Ic en l'acide diphosphonique correspondant, ou
un composé de formule IV, dans laquelle R⁵, R⁶, R⁷ et R⁸ ont les significations données dans la formule Ia,
E) avec un composé de formule IX 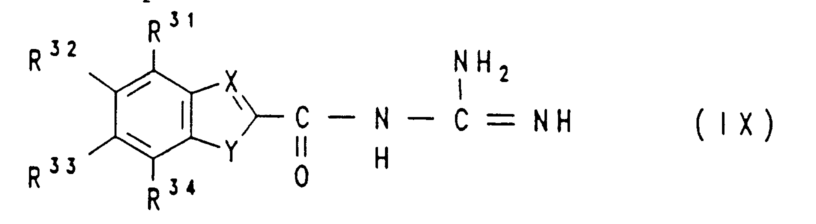 en présence d'un solvant inerte, pour obtenir un composé de formule Ia, Ib ou Ic, R³¹, R³², R³³ et R³⁴ ayant les significations données dans la formule Ia, Ib ou Ic, et éventuellement
F) on convertit l'ester d'acide diphosphonique du composé de formule Ia, Ib ou Ic en l'acide diphosphonique correspondant.

6. Médicament, contenant une quantité efficace d'au moins l'un des composés de formule tautomère Ia, Ib ou Ic selon la revendication 1 et/ou au moins l'un des sels physiologiquement acceptables d'un composé de formule tautomère Ia, Ib ou Ic, en plus d'adjuvants et/ou de véhicules pharmaceutiquement appropriés et physiologiquement acceptables, de diluants et/ou d'autres substances actives.

7. Procédé pour la fabrication d'un médicament selon la revendication 6, caractérisé en ce que l'on met sous une forme d'administration appropriée au moins un composé de formule tautomère Ia, Ib ou Ic et/ou au moins l'un des sels physiologiquement acceptables du composé de formule tautomère Ia, Ib ou Ic, éventuellement avec des adjuvants et/ou véhicules, diluants et/ou autres substances actives.

8. Utilisation d'un composé de formule Ia, Ib ou Ic selon une ou plusieurs des revendications 1 à 4 et/ou tels qu'obtenu conformément au procédé selon la revendication 5, pour la fabrication d'un médicament destiné à la prophylaxie ou au traitement de maladies dégénératives du système osseux.

9. Utilisation selon la revendication 8, pour la fabrication d'un médicament destiné au traitement de maladies à résorption osseuse accrue, en particulier la maladie de Paget, l'ostéocarcinome métastasique, l'hypercalcémie ou l'ostéoporose.
